(19) Europäisches Patentamt
European Patent Office
Office européen des brevets

(11) EP 0 358 154 B1

(12) **EUROPEAN PATENT SPECIFICATION**

(45) Date of publication and mention of the grant of the patent:
**02.12.1998 Bulletin 1998/49**

(51) Int. Cl.[6]: **C07D 473/00**, C07D 239/46, C07D 239/54, A61K 31/52, A61K 31/505

(21) Application number: **89116327.1**

(22) Date of filing: **05.09.1989**

# (54) Novel cyclobutane derivative and process for producing same

Cyclobutanderivat und Verfahren zu seiner Herstellung

Dérivé de cyclobutane et procédé pour sa production

(84) Designated Contracting States:
**BE DE ES FR GB IT NL SE**

(30) Priority: **09.09.1988 JP 224476/88**
**22.11.1988 JP 295135/88**
**27.02.1989 JP 43036/89**
**22.06.1989 JP 158223/89**

(43) Date of publication of application:
**14.03.1990 Bulletin 1990/11**

(73) Proprietor:
**NIPPON KAYAKU KABUSHIKI KAISHA**
**Tokyo 102 (JP)**

(72) Inventors:
- **Ichikawa, Yuh-ichiro**
**Tokyo (JP)**
- **Narita, Aya**
**Ageo-shi (JP)**
- **Matsuo, Kaoru**
**Kashiwa-shi (JP)**
- **Aoyama, Keiko**
**Tokyo (JP)**
- **Matsumura, Fumiko**
**Urayasu-shi (JP)**
- **Nishiyama, Yukihiro**
**Aichi-gun Aichi-ken (JP)**
- **Matsubara, Kenichi**
**Suita-shi (JP)**
- **Nagahata, Takemitsu**
**Toyonaka-shi (JP)**
- **Hoshino, Hiroo**
**Maebashi-shi (JP)**
- **Seki, Jun-ichi**
**Takasaki-shi (JP)**
- **Narasaka, Koichi**
**Tokyo (JP)**
- **Hayashi, Yujiro**
**Tokyo (JP)**

(74) Representative:
**Türk, Gille, Hrabal**
**Brucknerstrasse 20**
**40593 Düsseldorf (DE)**

(56) References cited:

| | |
|---|---|
| **EP-A- 0 159 264** | **EP-A- 0 184 473** |
| **EP-A- 0 291 229** | **EP-A- 0 335 355** |
| **EP-A- 0 366 059** | **US-A- 4 177 348** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 358 154 B1

## Description

BACKGROUND OF THE INVENTION

1. Field of the Invention

This invention relates to cyclobutane derivatives expectedly useful as medical drugs such as antiviral agent, carcinostatic agent and the like, as well as to their useful production intermediates and a process for producing them.

2. Description of the Prior Art

Many of the nucleic acid-related substances are known to have an antiviral activity or a carcinostatic activity, and some of them are clinically used as useful medical drugs. For instance, Vidarabine (M. Privat de Garilhe and J. de Rubber, C. R. Acad. Soc. D (Paris) 259, 2725 (1964)), Aciclovir (G. B. Elion et al., Proc. Natl. Acad. Sci. USA, 74, 5716 (1977) and Azidothymidine (H. Mitsuya et al., Proc. Natl. Acad. Sci. USA, 82, 7096 (1985) are known as antiviral agents, and 5-Fluorouracil and cytosine arabinoside are known as carcinostatic agents.

However, the above-mentioned antiviral agents are not widely applicable and limited in the method of administration because of their solubility, oral absorbability and influence on metabolism. Further, they have many problems such as difficulty of longterm administration because of side reactions such as bone marrow supression. Further, because of the increasing tendency of malignant viral diseases such as acquired immuno-deficiency syndrome (AIDS), T cell leukemia in the adult (ATL), etc., development of new excellent antiviral drug is desired.

On the other hand, the above-mentioned carcinostatic agents also have many unsolved problems regarding applicability and side reaction, etc.

SUMMARY OF THE INVENTION

This invention relates to (1R,2R,3S)cyclo-butane derivatives represented by the following general formula (IV):

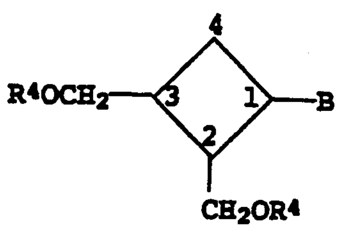

(IV)

wherein $R_4$ represents hydrogen atom (or - in case of a intermediate product - a conventionally used protecting group) and B represents a pyrimidine base represented by

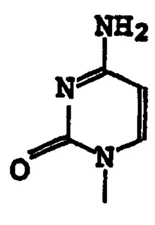

or

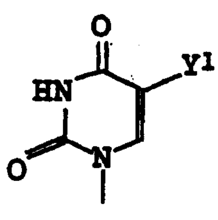

or a purine base represented by

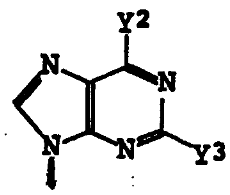

or

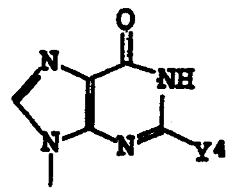

wherein $Y^1$ represents hydrogen atom or $C_{1-4}$ lower alkyl group; $Y^2$ represents hydrogen atom, halogen atom or amino group; $Y^3$ represents hydrogen atom or amino group; and $Y^4$ represents hydrogen atom or amino group.

DETAILED DESCRIPTION OF THE INVENTION

Concrete examples of the compound represented by general formula (IV) are shown below.

1. 9-[(1R,2R,3S)-2,3-Bis(hydroxymethyl)cyclobutane-1-yl] -adenine
2. 9-[(1R,2R,3S)-2,3-Bis(hydroxymethyl)cyclobutane-1-yl] -guanine
3. 2-Amino-9-[(1R,2R,3S)-2,3-bis(hydroxymethyl)cyclobutane-1-yl] -purine
4. 2-Amino-9-[(1R,2R,3S)-2,3-bis(hydroxymethyl)cyclobutane-1-yl] -6-chloropurine
5. 2,6-Diamino-9-[(1R,2R,3S)-2,3-bis(hydroxymethyl)cyclobutane-1-yl]-purine
6. 9-[(1R,2R,3S)-2,3-Bis(hydroxymethyl)cyclobutane-1-yl] -hypoxanthine
7. 1-[(1R,2R,3S)-2,3-Bis(hydroxymethyl)cyclobutane-1-yl] -2,4(1H,3H)-pyrimidindione
8. 1-[(1R,2R,3S)-2,3-Bis(hydroxymethyl)cyclobutane-1-yl] -5-methyl-2,4(1H,3H)-pyrimidindione
9. 4-Amino-1-[(1R,2R,3S)-2,3-bis(hydroxymethyl)cyclobutane-1-yl]-2(1H)-pyrimidinone
10. 2-Amino-9-[(1R, 2R, 3S)-2,3-bis(acetoxymethyl) cyclobutane-1-yl]-purine

Among the compounds of this invention, the compounds represented by general formula (IV) can be produced by reacting a compound represented by the following general formula (V):

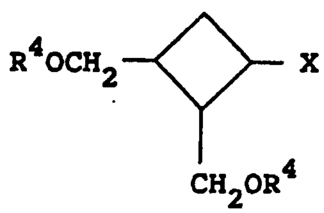

(V)

($R^4$ represents hydrogen atom or a protecting group and X represents a leaving group) with a nucleic acid base to obtain a compound represented by the following general formula (VI):

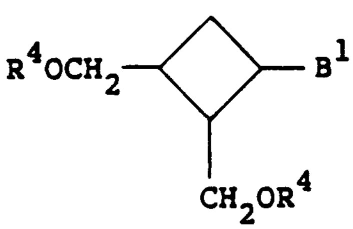

(VI)

($R^4$ represents hydrogen atom or a protecting group and $B^1$ represents a nucleic acid base) and, when the latter compound has a protecting group, optionally eliminating the protecting group with an appropriate protecting group-eliminating reagent.

As the protecting group $R^4$ in general formula (V) and (VI), any group may be used without limitation so far as it is conventionally used as a protecting group. Examples of the protecting group $R^4$ include ester type protecting groups such as acyl groups (e.g. acetyl, benzoyl and the like) and carbamoyl groups (e.g. dimethylcarbamoyl, diphenylcarbamoyl and the like), ether type protecting groups such as silyl groups (e.g. t-butyldimethylsilyl, t-butyldiphenylsilyl and the like), ($C_1$-$C_4$) alkoxy-($C_1$-$C_4$) alkyl groups (e.g. methoxymethyl and the like), tetrahydropyranyl group, benzyl groups (e.g. benzyl group, 4-methoxybenzyl group, trityl group and the like). Examples of the leaving group X in general formula (V) include sulfonyloxy groups such as methanesulfonyloxy, p-toluenesulfonyloxy, trifluoromethanesulfonyloxy and the like, and halogen atoms such as chlorine, bromine, iodine and the like.

Examples of the nucleic acid base include pyrimidine bases such as uracil, thymine, cytosine, 5-fluorouracil and the like, and purine bases such as adenine, hypoxanthine, guanine, 2-amino-6-chloropurine, 2-aminopurine, 2,6-diaminopurine and the like. These compounds may have a protecting group. For example, the compounds represented by the following general formulas (XII) to (XVII) can be referred to:

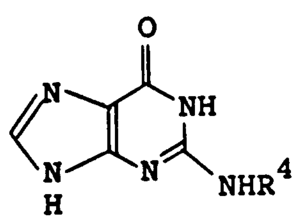

(XII)

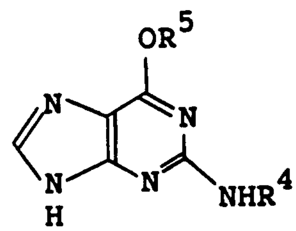

(XIII)

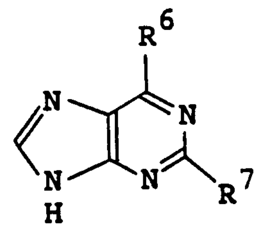

(XIV)

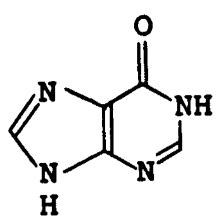

(XV)

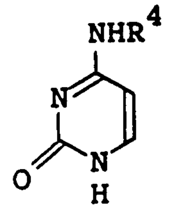

(XVI)

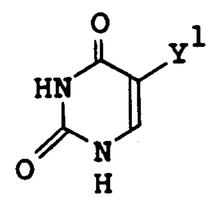

(XVII)

(in these formulas, $R^4$ is as defined above; $R^5$ represents $C_1$-$C_5$ lower alkyl group such as benzyl, butyl and the like, ($C_1$-$C_5$ alkoxy)-($C_1$-$C_5$ alkyl) group such as methoxyethyl and the like, or $R^4$; $R^6$ represents hydrogen atom, halogen atom or $NHR^4$; $R^7$ represents hydrogen atom or $NHR^4$; and $Y^1$ represents hydrogen atom, halogen atom or $C_1$-$C_5$ lower alkyl group).

In the reaction between a compound represented by general formula (V) and a nucleic acid base (for example, a compound of general formula (XII)-(XVII)), the ratio between the compound of general formula (V) and the compound of general formula (XII)-(XVII) is recommendably about 0.5 to about 10 equivalents and more preferably about 1 to about 5 equivalents of the latter compound per 1 equivalent of the former compound. This reaction is carried out either in the presence of a basic catalyst or in the absence of catalyst. As basic catalyst, potassium carbonate, lithium hydride, sodium hydride and the like can be used. The reaction is carried out in a solvent such as N,N-dimethylformamide (DMF), dimethyl sulfoxide (DMSO), 1,3-dimethyl-2-imidazolinone, hexamethyl phosphoric triamide (HMPA) and the like, at a temperature ranging from 0°C to reflux temperature of the solvent, preferably from ambient temperature to about 170°C. Based on the amount of the compound of general formula (XII)-(XVII), the basic catalyst is recommendably used in an amount of 0 to 2 equivalents, preferably 0.5 to 1.5 equivalents, and more preferably about 0.8 to 1.2 equivalents.

Elimination of the protecting group (including alkyl group) from the compound of general formula (VI) can be achieved by using appropriate protecting group-eliminating reagent and protecting group-eliminating method in accordance with the kind of protecting group. As the protecting group-eliminating reagent, alkalis such as sodium hydroxide, sodium methylate, ammonia and the like, acids such as hydrochloric acid, sulfuric acid and the like, fluorinated reagent such as tetrabutylammonium fluoride, and the like can be referred to, for example. As the protecting group-eliminating

method, hydrogenolysis and the like can be referred to, for example.

Among the compounds of this invention represented by general formula (IV), those of which B is 2,6-diaminopurine, 2-aminopurine or 2-amino-6-halopurine can be produced from a compound represented by the following general formula (IVa), too:

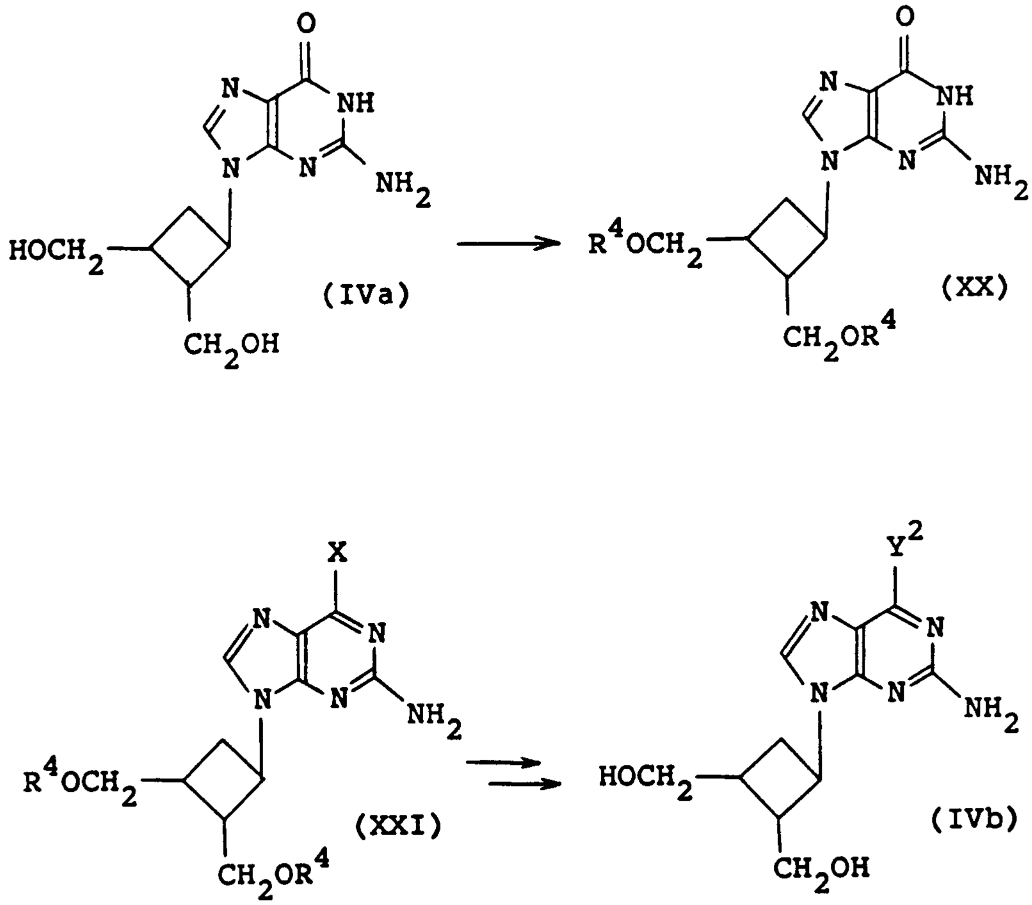

(IVa) (XX) (XXI) (IVb)

Reaction Scheme (1)

(in this scheme, $R^4$ is as defined above; X represents a leaving group; and $Y^2$ represents hydrogen atom, halogen atom or amino group).

For example, as shown in Reaction Scheme (1), hydroxyl group of a guanine derivative represented by general formula (IVa) is protected to prepare a compound represented by general formula (XX), after which it is reacted with a halogenating agent such as phosphorus oxychloride and the like or a sulfonylating agent such as 1,3,5-trimethylbenzenesulfonyl chloride and the like to synthesize a compound represented by general formula (XXI). The compound (XXI) thus obtained is heated in ampoule together with ammonia-methanol to give a compound of general formula (IVb) wherein $Y^2$ is amino group. If this compound is catalytically reduced by the use of palladium-carbon catalyst and thereafter the protecting group is eliminated therefrom, there is obtained a compound of general formula (IVb) wherein $Y^2$ is hydrogen atom. Otherwise, by eliminating protecting group from a compound of general formula (XXI) wherein X is halogen atom, there is obtained a compound of general formula (IVb) wherein $Y^2$ is halogen atom.

The compounds of this invention represented by general formula (IV) wherein B is hypoxanthine can be produced from a compound represented by the following general formula (IVc), too:

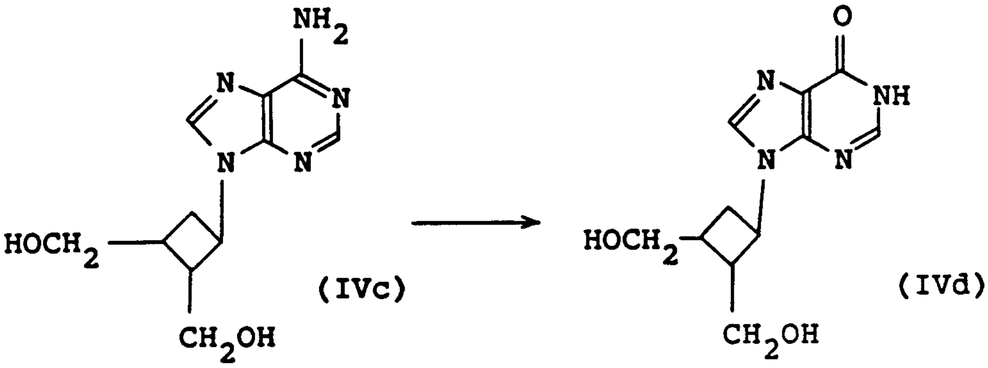

(IVc) (IVd)

**Reaction Scheme (2)**

Thus, as shown in Reaction Scheme (2), an adenine derivative of general formula (IVc) is diazotized by, for example, a treatment with nitrous acid and thereafter hydrolyzed or treated with a hydrolyzing enzyme such as Adenosine deaminase or the like, whereby a compound represented by general formula (IVd) can be obtained.

Further, the compounds of general formula (IV) can be synthesized in the following manner. Thus, as shown in the following Reaction Scheme (3):

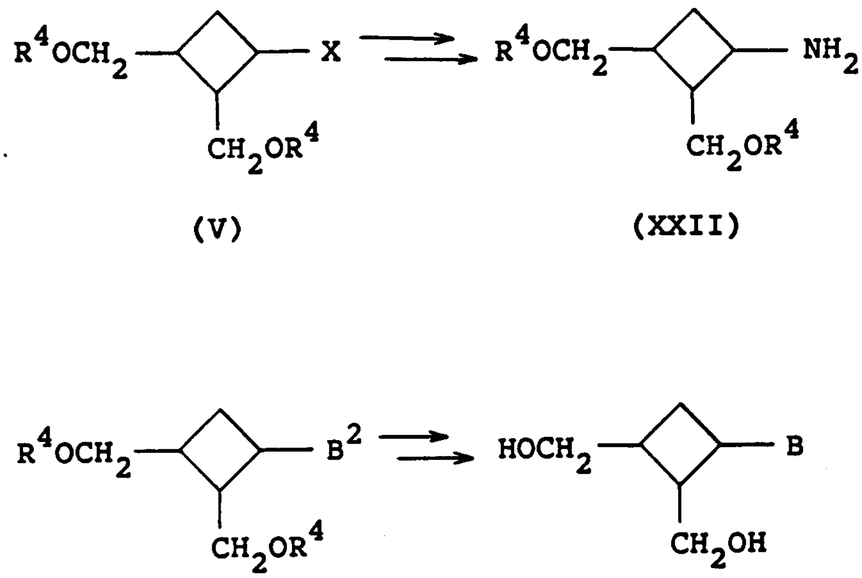

(V) (XXII)

(XXIII) (IV)

(in this reaction scheme, $R^4$ represents hydrogen atom or a protecting group, X represents an leaving group, B represents a nucleic acid base, and $B^2$ represents a substituent convertible to B in one step or plural steps) a compound of general formula (V) is treated with an azide ion compound such as sodium azide or the like and thereafter reduced in the usual way to synthesize an amine derivative represented by general formula (XXII), and the latter (XXII) is converted to a compound of general formula (IV) via an intermediate represented by general formula (XXIII) according to the known method (R. Vince et al., J. Med. Chem., 27, 1358 (1984); R. Vince et al., J. Med. Chem., 30, 2026 (1987); Y. F. Shealy and C. A. O'Dell, J. Heterocyclic Chem., 13, 1015, (1976); Y. F. Shealy et al., J. Heterocyclic Chem., 18, 383 (1981); etc.).

Further, it was found that a compound represented by general formula (III) is very useful for the production of a compound of general formula (V) or a compound of this invention represented by general formula (IV). Since the compounds of general formula (III) can be obtained in an optically active form, the compounds of general formula (IV) are also obtainable in an optically active form.

Thus, as shown in the following Reaction Scheme (4):

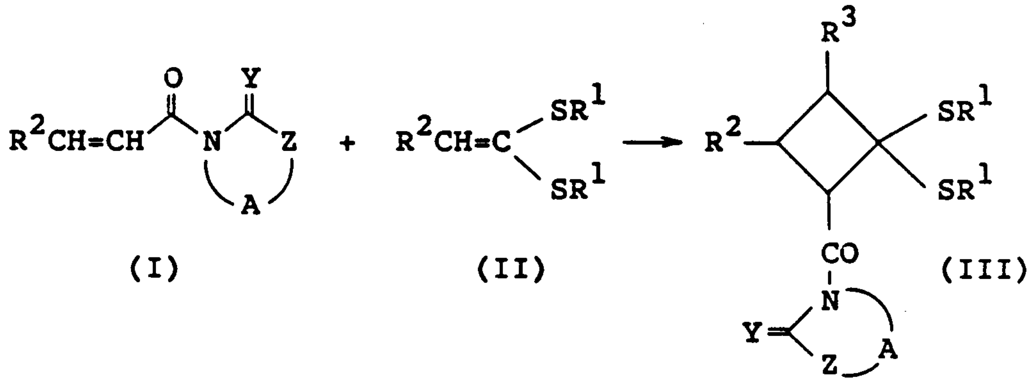

Reaction Scheme (4)

(in this reaction scheme, $R^1$, taken individually, represents an alkyl group having 1 to 5 carbon atoms or an aralkyl group or, taken in conjunction of two $R^1$ groups, represents a cyclic alkylene group having 2 to 3 carbon atoms; $R^2$ represents hydrogen atom, alkyl group having 1 to 5 carbon atoms, protected hydroxyalkyl group or protected carboxyl group; $R^3$ represents hydrogen atom, lower alkyl group having 1 to 5 carbon atoms, lower alkoxy group having 1 to 5 carbon atoms or aralkyloxy group; A represents a straight or branched chain alkylene group having 2 to 5 carbon atoms; Y represents oxygen atom or sulfur atom; and Z represents substituted or unsubstituted methylene group, oxygen atom or sulfur atom), a compound of general formula (I) and a compound of general formula (II) are reacted in the presence of a condensation catalyst, whereby a cyclobutane compound represented by general formula (III) is obtained in a high yield and this product is racemic or optically active in accordance with the kind of the catalyst. As the condensation catalyst usable in this reaction, Lewis acids and combinations of a Lewis acid and an equivalent or excessive amount of a ligand can be referred to, for example. Examples of said Lewis acid include titanium compounds such as titanium tetrachloride, dichlorodiisopropoxytitanium and the like, tin compounds such as stannous chloride, stannic chloride, stannous trifluoromethansulfonate or the like, and aluminum compounds such as dimethylaluminum chloride, diethylaluminum chloride and the like. As said ligand, sterically complicated diols are preferable. Examples of said ligand include compounds having a ring not smaller than 5-membered ring (preferably 5- to 8-membered ring) in molecule and two hydroxy-containing groups in both sides of the ring, such as (2S,3S)-2,3-O-(1-phenylethylidene)-1,1,4,4-tetraphenyl-1,2,3,4-butanetetraol (Compound A), (2R,3R)-2,3-O-(1-phenylethylidene)-1,1,4,4-tetraphenyl-1,2,3,4-butanetetraol (Compound B), (2S,3S)-2,3-O-benzylidene-1,1,4,4-tetraphenyl-1,2,3,4-butanetetraol (Compound C), (2R,3R)-2,3-O-benzylidene-1,1,4,4-tetraphenyl-1,2,3,4-butanetetraol (Compound D), (2S,3S)-2,3-O-(1-phenylethylidene)-1,1,4,4-tetrakis(4-methoxyphenyl)-1,2,3,4-butanetetraol (Compound E), (2R,3R)-2,3-O-(1-phenylethylidene)-1,1,4,4-tetrakis(4-methoxyphenyl)-1,2,3,4-butanetetraol (Compound F), racemic forms of the above-mentioned compounds, and the like. The compound of general formula (I) and the compound of general formula (II) are used in such a proportion that the amount of compound (II) is 0.1 to 5 equivalents, preferably 0.5 to 2 equivalents, per one equivalent of compound (I). The condensation catalyst is used in an amount of 0.001 to 2 equivalents, preferably 0.01 to 1.2 equivalents, per one equivalent of the compound of general formula (I). This reaction can sometimes be made to progress more efficiently by adding a dehydrating agent such as Molecular Sieves 4A and the like into the reaction system. Examples of the solvent usable in this reaction include hydrocarbon solvents such as pentane, hexane, heptane, petroleum ether, benzene, toluene, ethylbenzene, trimethylbenzene, triisopropylbenzene and the like; halogenated hydrocarbon solvents such as Flon and the like; ethereal solvents such as ether, tetrahydrofuran and the like; acetonitrile; and mixtures of these solvents. The reaction temperature is recommendably in the range from freezing point of reaction solvent to its boiling point, and preferably in the range of -50°C to about 30°C. For example, by reacting one equivalent of a compound of general formula (I) wherein $R^2$ is methoxycarbonyl group, A is $CH_2CH_2$, Y is oxygen and Z is oxygen with 1.25 equivalents of a compound of general formula (II) wherein $R^1$ is methyl group and $R^3$ is hydrogen atom in the presence of a condensation catalyst (combination of 0.05 equivalent of dichlorodiisopropoxytitanium and 0.055 equivalent of (2S,3S)-2,3-O-(1-phenylethylidene)-1,1,4,4-tetraphenyl-1,2,3,4-butanetetraol (Compound A)) and Molecular Sieves

4A, in a solvent mixture consisting of hexane and toluene at a reaction temperature of 0°C, (2S,3S)-3-methoxycarbonyl-1,1-bis(methylthio)-2-(oxazolidin-2-one-3-yl)-carbonylcyclobutane is obtained in a high chemical yield and a high optical yield. If this reaction is carried out in the presence of a combined catalyst consisting of dichlorodiisopropoxytitanium and (2R,3R)-2,3-O-(1-phenylethylidene)-1,1,4,4-tetraphenyl-1,2,3,4-butanetetraol (Compound B), (2R,3R)-3-methoxycarbonyl-1,1-bis(methylthio)-2-(oxazolidin-2-one-3-yl)-carbonylcyclobutane is obtained. Further, if this reaction is carried out by using racemic 2,3-0-(1-phenylethylidene)-1,1,4,4-tetraphenyl-1,2,3,4-butanetetraol, (±)-(2α,3β)-3-methoxycarbonyl-1,1-bis(methylthio)-2-(oxazolidin-2-one-3-yl)-carbonylcyclobutane is obtained.

In the compound of this invention, examples of the alkyl group having 1 to 5 carbon atoms represented by $R^1$, $R^2$ and $R^3$ include alkyl groups such as methyl, ethyl, butyl and the like; examples of the aralkyl group include alkyl groups substituted by an aromatic ring such as benzyl group, 4-methoxybenzyl group and the like; examples of the protected hydroxyalkyl group include benzyloxymethyl group, acetyloxymethyl group, t-butyldiphenylsilyloxymethyl group and the like; examples of the protected carboxyl group include alkoxycarbonyl groups such as methoxycarbonyl, ethoxycarbonyl and the like and aralkyloxycarbonyl groups such as benzyloxycarbonyl and the like; examples of the alkoxy group having 1 to 5 carbon atoms include methoxy group, allyloxy group and the like; and examples of the aralkyloxy group include benzyloxy group, 4-methoxybenzyloxy group, t-butyldiphenylsilyloxy group and the like.

In this invention, examples of the compound represented by general formula (III) include the compounds of general formula (III) wherein $R^1$, $R^2$, $R^3$, A, Y and Z are as shown in Table 1:

Table 1

| No | $R^1$ | $R^2$ | $R^3$ | A | Y | Z |
|---|---|---|---|---|---|---|
| 13 | Me | COOMe | H | $CH_2CH_2$ | O | O |
| 14 | Me | COOBn | H | $CH_2CH_2$ | O | O |
| 15 | Me | H | H | $CH_2CH_2$ | O | O |
| 16 | Me | COOMe | H | $CH_2CH_2$ | S | S |
| 17 | Me | H | OBn | $CH_2CH_2$ | O | O |

provided that the two $R^1$ groups in the compound of general formula (III) may be identical or different from each other.

The compounds represented by general formula (V) can be produced, for example, from a compound represented by general formula (III) (herein $R^2$ is a protected carboxyl group and $R^3$ is hydrogen atom) according to a process represented by the following Reaction Scheme (5):

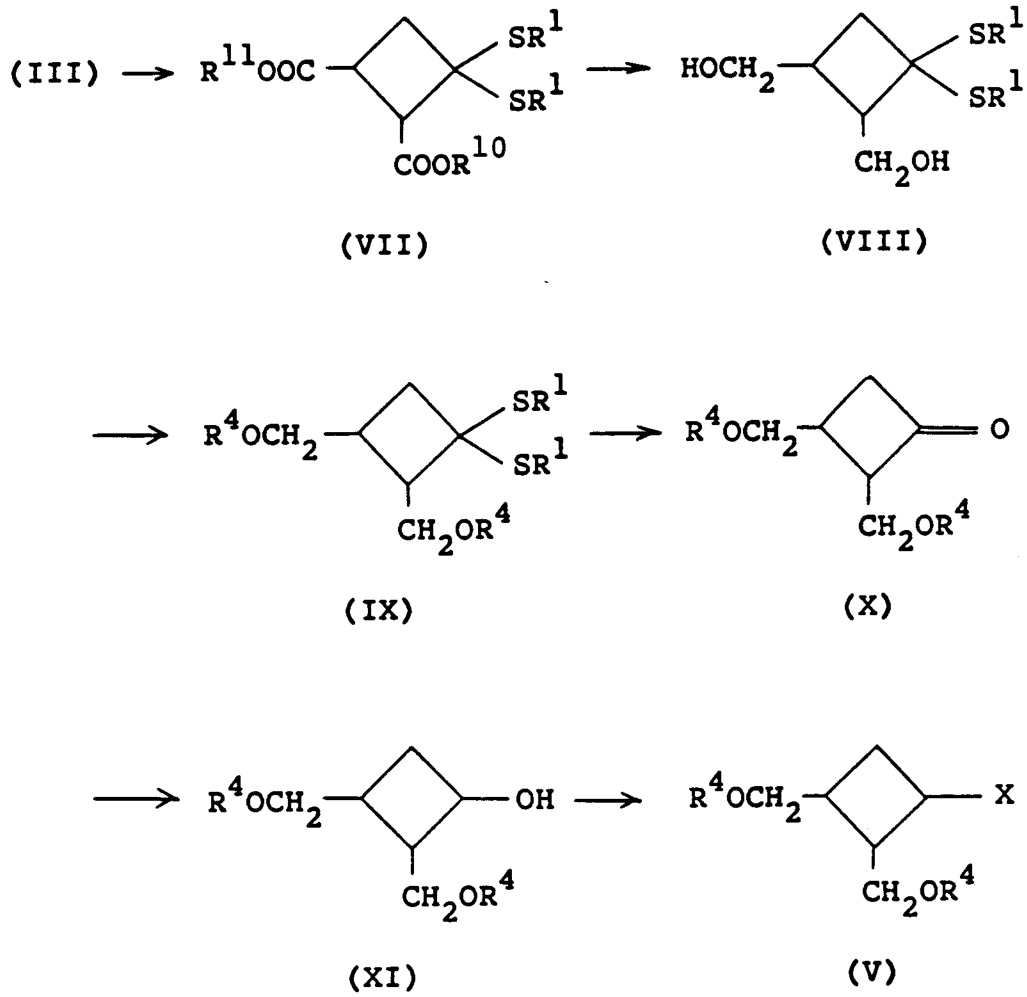

(III) (VII) (VIII) (IX) (X) (XI) (V)

Reaction Scheme (5)

(in this reaction scheme, $R^1$ is the same as in general formula (III), $R^{10}$ and $R^{11}$ each represents hydrogen atom, alkyl group having 1 to 5 carbon atoms or aralkyl group, $R^4$ represents hydrogen atom or a protecting group, and X represents an eliminable group).

Thus, in the first step, a compound represented by general formula (III) (herein, $R^2$ is a protected carboxyl group and $R^3$ is hydrogen atom) is reacted in an alcoholic solvent such as methanol, ethanol or the like in the presence of a corresponding metallic alkoxide such as magnesium methoxide, sodium ethoxide or the like at a temperature ranging from -78°C to boiling point of the solvent (preferably at a temperature not higher than room temperature), or solvolyzed in an alcoholic solvent such as methanol, ethanol or the like in the presence of an acid such as hydrochloric acid, p-toluenesulfonic acid and the like or a base such as triethylamine, sodium hydroxide and the like, whereby the corresponding ester represented by general formula (VII) wherein $R^{10}$ and $R^{11}$ represent hydrogen atom, alkyl group having 1 to 5 carbon atoms or aralkyl group can be obtained. Otherwise, it is hydrolyzed in the presence of an acid such as hydrochloric acid, p-toluensulfonic acid and the like or a base such as sodium hydroxide, potassium carbonate and the like, whereby a carboxylic acid of general formula (VII) wherein $R^{10}$ and $R^{11}$ represent hydrogen atom can be obtained. By reducing the compound of general formula (VII) obtained herein with a metal hydride such as lithium aluminum hydride, di(isobutyl)-aluminum hydride, sodium boron hydride, diborane and the like, an alcohol represented by general formula (VIII) is obtained. Next, the hydroxyl group of the compound of general formula (VIII) is protected to obtain a compound of general formula (IX), and its dithioketal part is hydrolyzed in an aqueous solvent with a halogenating agent such as iodine, N-bromosuccinimide, N-chlorosuccinimide, sulfuryl chloride and the like or a heavy metal compound such as silver nitrate, silver oxide, silver perchlorate, mercury chloride, copper chloride, copper oxide and the like or a

combination of these compounds to form a ketone compound represented by general formula (X).

A compound represented by general formula (X) is reduced with a metal-hydrogen complex compound such as lithium aluminum hydride, lithium tri(t-butoxy)-aluminum hydride, sodium boron hydride, lithium tri(s-butyl)-boron hydride, lithium boron hydride and the like or a metal hydride such as di-isobutyl-aluminum hydride, diborane and the like as a reductant, in a solvent such as hydrocarbon type solvent (e.g. pentane, hexane, heptane, petroleum ether, benzene, toluene, ethylbenzene and the like), halogenated hydrocarbon type solvent (e.g. methylene chloride, chloroform and the like), ethereal solvent (e.g. ether, tetrahydrofuran and the like), alcoholic solvent (e.g. methanol, ethanol and the like), water, or mixture thereof, at a reaction temperature of -100°C to 50°C and preferably -80°C to 30°C, to give a compound represented by general formula (XI). In this reduction, one of the stereoisomers can be preferentially obtained by selecting the kind of reductant and the conditions of reaction. For example, if 2,3-trans cyclobutanone represented by general formula (Xa) shown in the following Reaction Scheme (6):

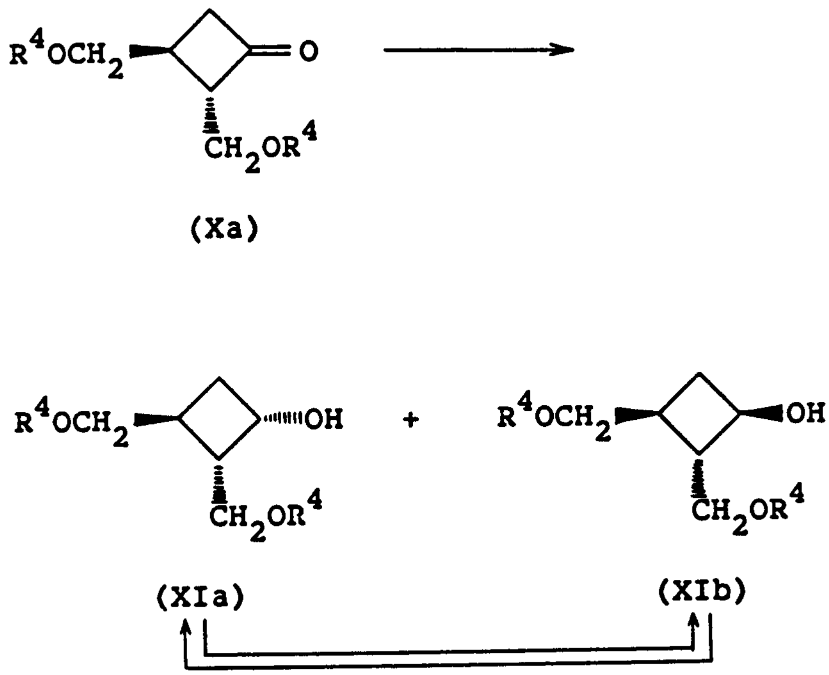

(Xa)

(XIa) (XIb)

Reaction Scheme (6)

(in this scheme, $R^4$ represents hydrogen atom or a protecting group) is reduced with a sterically relatively simple reductant, a 1,2-trans alcohol represented by general formula (XIb) is selectively formed. However, if the reduction is carried out with a greatly sterically hindered reductant, a 1,2-cis alcohol represented by general formula (XIa) is preferentially formed. For instance, if (2S,3S)-2,3-bis(t-butyldiphenylsilyloxymethyl)-1-cyclobutanone is reduced with lithium tri(t-butoxy)-aluminum hydride or sodium boron hydride (both these reductants are not greatly hindered sterically), (1R,2S,3S)-2,3-bis(t-butyldiphenylsilyloxymethyl)-cyclobutanol is selectively formed (isolation yield 88% and 80%, respectively). If the same starting compound as above is reduced with lithium tri(s-butyl)-boron hydride or di(isobutyl)-aluminum hydride (both these reductants are greatly hindered sterically), (1S,2S,3S)-2,3-bis(t-butyldiphenylsilyloxymethyl)-cyclobutanol is preferentially formed (isolation yield 75% and 82%, respectively). The results obtained are as shown in Table 2.

Table 2

| Conditions | | | Yield (%) | |
|---|---|---|---|---|
| Reagent | Solvent | Temp., °C | XIa | XIb |
| $LiAl(OtBu)_3H$ | THF | -78-RT | 9 | 88 |
| $NaBH_4$ | $THF-H_2O$ | RT | 19 | 80 |
| $Li(sBu)_3BH$ | THF | -78 | 75 | 23 |
| $(iBu)_2AlH$ | $CH_2Cl_2$ | -78 | 74 | 22 |
| $(iBu)_2AlH$ | Toluene | -78 | 82 | 17 |
| $R^4$ = t-$BuPh_2Si$ (in Xa, XIa and XIb) | | | | |

The stereoisomers obtained herein, i.e. the compound of general formula (XIa) and the compound of general formula (XIb), after isolation, are both easily convertible from one to the other. For example, a compound represented by general formula (XIa) or a compound represented by general formula (XIb) is again oxidized to a ketone represented by general formula (Xa) by means of conventional oxidant or oxidizing method (for example, metal oxidizing agent such as chromic acid/acetic acid, chromic acid/pyridine and the like, or dimethyl sulfoxide (DMSO)-oxidation method such as DMSO-acetic anhydride/acetic acid, DMSO-oxalyl chloride-triethylamine/methylene chloride and the like), after which the oxidized product (Xa) is subjected to a selective reduction. Otherwise, steric configuration of 1-carbon atom to which hydroxyl group is linked in a compound of general formula (XIa) or a compound of general formula (XIb) is inverted by utilizing Mitsunobu Reaction, for example. Now, the Mitsunobu Reaction in the latter method will be explained below. Thus a compound represented by general formula (XIa) or (XIb) is reacted with a trivalent phosphorus compound such as triphenylphosphine, trimethyl phosphite, triethyl phosphite or the like, a carboxylic acid such as acetic acid, benzoic acid or the like, and an azodicarboxylic ester such as diethyl azodicarboxylate or the like, in a solvent such as hydrocarbon type solvent (e.g. pentane, hexane, heptane, petroleum ether, benzene, toluene, ethylbenzene and the like), halogenated hydrocarbon solvent (e.g. methylene chloride, chloroform and the like), ethereal solvent (e.g. ether, tetrahydrofuran and the like) or mixture thereof, at a reaction temperature of -100°C to 50°C (preferably -50°C to 30°C) to form an ester. Then, the ester is hydrolyzed with an alkali such as potassium carbonate, sodium hydroxide, sodium methylate, ammonia and the like or an acid such as hydrochloric acid, sulfuric acid and the like or reduced with a metal-hydrogen complex compound such as lithium aluminum hydride, lithium tri(s-butyl)-boron hydride, lithium boron hydride and the like or a metal hydride such as di-isobutyl-aluminum hydride, diborane and the like. By this treatment, a compound represented by general formula (XIb) or a compound represented by general formula (XIa) can be obtained. For instance, if (1R,2S,3S)-2,3-bis(t-butyldiphenylsilyloxymethyl)cyclobutanol is reacted with triphenylphosphine benzoic acid diethyl azodicarboxylate in benzene to form (1S,2S,3S)-1-benzoyl-2,3-bis(t-butyldiphenylsilyloxymethyl)cyclobutane and then the latter is reduced with di-isobutylaluminum hydride in toluene, the benzoyl group can be eliminated, and (1S,2S,3S)-2,3-bis(t-butyldiphenylsilyloxymethyl)cyclobutanol is obtained. By converting the secondary hydroxyl group of the compound represented by general formula (XI) thus obtained into an leaving group, a compound represented by general formula (V) can be produced.

The experimental examples presented below will demonstrate that the compounds of this invention exhibit wide and strong antiviral and carcinostatic activities.

Experimental Example 1

Antiviral activities against simple Herpes 1 type virus (HSV-I) and simple Herpes 2 type virus (HSV-II), both belonging to DNA virus, were examined by the following methods.

(Method 1)

A 6-well multi-plate having a single layer of Vero cell (derived from the kidney cell of African Green-Monkey was infected with 100 to 150 PFU (plaque forming units) of virus. After adsorption at 37°C for one hour, a layer of agar medium (Eagle MEM medium containing 1.5% of agar) containing a varied concentration of sample was superposed thereon, and a cultivation was carried out at 37°C for 48 hours in 5% (v/v) carbon dioxide incubator. The formation of plaque was measured, from which 50% inhibitory value ($IC_{50}$) was determined.

Experimental Example 2

Antiviral activity against human Cytomegalovirus (HCMV) belonging to DNA virus was examined by the following method.

(Method 2)

Anti-HCMV activity was determined in the following manner. Thus, a 35 mm dish having a single layer of human embryonal fibroblast was infected with 100 PFU of HCMV (A0169 strain). After adsorption for one hour, one layer of a medium (0.5% agarose, 2% fetal calf serum) containing a varied concentration of sample compound was superposed thereon, and cultivation was carried out at 37°C for 10 days in 5% (v/v) carbon dioxide incubator. Then, formation of plaque was measured, from which 50% inhibitory value ($IC_{50}$) was determined.

Experimental Example 3

Antiviral activity against B hepatitis virus (HBV) belonging to DNA virus was examined by the following method.

(Method 3)

According to the procedure of Dulbecco (Proc. Natl. Acad. Sci. USA, 84, 444 (1987)), cultured liver cell strain HB611 producing and releasing active B hepatitis virus was cultured in a modified Eagle medium (GIBCO) at 37°C at a $CO_2$ concentration of 5% in the presence of 10% fetal calf serum, 200 micrograms/ml of G418, 100 μ/ml of Penicillin and 100 μ/ml of Streptomycin. The culture fluid was inoculated onto a 6-well plate at a rate of 5 x $10^4$ cells/well (35 mm). One or two days after, 50% confluence was reached, when a predetermined quantity of sample compound was added and cultivation was continued. Thereafter, cultivation was continued for 15 days, while refreshing the medium with a fresh medium containing the same concentrations of the chemicals at intervals of 3 days. Then, the medium was removed, and the cell bodies were treated with 0.5 ml of Lysis buffer (10 mM Tris HCl, pH 7.8/5 mM $Na_2EDTA$, 1% SDS/0.1 mg/ml Pronase K) at 37°C for one hour and dissolved thereinto. The DNA thus obtained was purified by RNase treatment, phenolchloroform treatment and ethanol precipitation.

Then, 5 micrograms of DNA was subjected to Hind III treatment, and DNA pattern was analyzed according to Southern method by using $^{32}P$-labelled B hepatitis virus DNA as a probe.

Experimental Example 4

Antiviral activity against Human Immunodeficiency Virus (HIV) belonging to DNA virus was examined by the following method.

(Method 4)

MT-4 cell (about 50,000 cells/ml) was introduced into a 24-well tray, to which was added 100 micrograms of a solution containing a predetermined quantity of sample compound. Culture was carried out at 37°C for 2 hours in 5% (v/v) carbon dioxide incubator. Then, $10^3$ to $10^4$ infection units of HIV was added and cultured for 4 days, after which a part of the culture fluid was applied onto a slide glass and immobilized with acetone and development of virus antigen was tested by fluorescent antibody method.

As the primary antibody of fluorescent antibody method, serum of AIDS patient was used. As the secondary antibody, FITC-labelled human IgG was used.

Experimental Example 5

Carcinostatic activities on human cervical cancer HeLa $S_3$ cell, mouse leukemia L1210 cell and human leukemia P388 cell were examined by the following methods.

(Method 5)

HeLa $S_3$ cell was made into a suspension (7.5 x $10^3$ cells/ml). The suspension was spread on a 96-well flat plate at a rate of 0.2 ml/well. After culturing it at 37°C for 24 hours in 5% carbon dioxide incubator, 10 microliters of a sample was added and cultivation was carried out for 72 hours. Then, culture fluid was taken out from each well and immobilized with methanol, after which 0.1 ml/well of 0.05% Methylene Blue/10 mM-Tris-HCl (pH 8.5) solution was added and

dyeing was carried out at room temperature for 30 minutes. The dyed fluid in each well was withdrawn by means of aspirator and three times washed with pure water. Then, 3% HCl was added at a rate of 0.2 ml/well and the resulting mixture was sealed and allowed to stand at room temperature for about 24 hours, to extract pigment from the cells. Optical absorbance of each well at 660 nm was measured by means of Dynatic Microplate Reader, from which growth inhibitory rate (%) at varied concentration was calculated according to the following equation. The results were plotted on a logarithmic probability paper, from which 50% inhibitory concentration ($IC_{50}$, micrograms/ml) was determined.

$$\text{Growth inhibitory rate (\%)} = (1 - A_1/A_0) \times 100$$

wherein $A_1$ = Absorbance in the treated group,
$A_0$ = Absorbance in the control group.

(Method 6)

Cells of P388 and L1210 were spread onto a 24-well plate. After adding a sample, it was cultured at 37°C for 48 hours in 5% $CO_2$ incubator. After the culture, the number of cells was counted by means of coal tar counter. The growth inhibitory rate in the treated group, based on that in the control group, was calculated from the following equation, from which 50% inhibitory concentration ($CI_{50}$, micrograms/ml) was determined: The results obtained are as shown in Table 3.

$$\text{Growth inhibitory rate (\%)} = [1-(N_T-N_O)/N_C-N_O] \times 100$$

wherein $N_T$ = Cell number in treated group
$N_O$ = cell number at the start of culture
$N_C$ = Cell number in control group

Table 3

| Compound No. | $IC_{50}$(μg/ml) | | | | | | | |
|---|---|---|---|---|---|---|---|---|
| | HSV-1 | HSV-2 | HIV | HCMV | HBV | $HeLaS_3$ | P388 | L1210 |
| 1 | 7.3 | 8.9 | 0.03 | 12 | 0.024 | 14 | 0.58 | 1.1 |
| 2 | 0.03 | 0.13 | 0.3 | 0.4 | 0.86 | 19 | 1.6 | 8.1 |

Experimental Example 6

Activity of the sample (compound No. 2) against herpes simplex virus type 2 (HSV-2) infection was examined in mice. Balb/c mice (6 week-old, male) were inoculated intraperitoneally (I.p.) with HSV-2 strain 186 at $4.8 \times 10^5$ PFU/0.2 ml per mouse. The sample was formulated in saline and applied either orally (p.o.) or i.p. at the indicated doses once a day for 10 days, starting 6 hrs after virus infection. The mortality was monitored for 20 days after inoculation. Efficacy of the sample was indicated as extension of survival time and number of survivors. Aciclovir (ACV: commercialized antiviral agent) was used as reference sample.

Results:

The results obtained are as shown in Table 4.

Table 4

| Compound | Route | Dose (mg/kg/day) | Means survival time (day) | Survivors/Total |
|---|---|---|---|---|
| Control | | | 7.7 | 0/10 |

Table 4 (continued)

| Compound | Route | Dose (mg/kg/day) | Means survival time (day) | Survivors/Total |
|---|---|---|---|---|
| No. 2 | i.p. | 20 | 20.0 | 10/10 |
| | | 10 | 20.0 | 10/10 |
| | | 5 | 20.0 | 10/10 |
| | | 2.5 | 18.9 | 9/10 |
| | | 1.25 | 19.8 | 7/10 |
| | p.o. | 20 | 19.7 | 9/10 |
| | | 10 | 19.8 | 9/10 |
| | | 5 | 18.9 | 8/10 |
| | | 2.5 | 14.7 | 4/10 |
| | | 1.25 | 12.4 | 2/10 |
| ACV | i.p. | 100 | 5.4 | 2/10 |
| | | 50 | 16.1 | 5/10 |
| | | 25 | 12.7 | 1/10 |
| | | 12.5 | 11.1 | 1/10 |
| | p.o. | 100 | 14.3 | 2/10 |
| | | 50 | 14.8 | 4/10 |
| | | 25 | 11.1 | 0/10 |
| | | 12.5 | 9.3 | 0/10 |

To evaluate in vivo potential of compound No. 2, it was compared with ACV. Compound No. 2 was highly effective in reducing mortality rate of mice infected with HSV-2 irrespective of administration routes (Table 4). Mean survival time of mice in a control group was 7.7 days, while a number of survivors through an observation period of 20 days was observed in a wide doses range of compound No. 2 from 1.25 to 20 mg/kg per day. Survivors were also observed by the treatment of ACV. However, number of the survivors and an effective dose range were much greater with compound No. 2 than with ACV.

Having a strong antiviral activity, the compounds of this invention represented by general formula (IV) are expected to be effectively usable for controlling a number of viral diseases such as herpes labialis, herpes in the genital organs, herpes zoster, simple infection from Herpesvirus 1 and 2 (HSV-I, II), Varicells zoster virus (VZV), Cytomegalovirus (CMV) and Epstein-Barr virus (EBV), viral hepatitis, viral respiratory diseases, viral diseases of the digestive organs, AIDS, ATL, etc. Further, since they have a carcinostatic activity, they are expectedly usable as a carcinostatic agent.

In using the compounds of this invention obtained in the above-mentioned manner as an antiviral or carcinostatic drug, they can be administered orally, intravenously or percutaneously to the warm-blood animal. Though the dose may vary dependent on symptoms and age of the warm-blood animal and the method of administration, it is usually 0.1 to 500 mg/kg/day. The compounds of this invention are administered in the form of a composition prepared by mixing them with appropriate excipients. As the form of composition, tablet, granule, powder, capsule, injection, cream, suppository, and the like can be used.

Next, production of the compounds of this invention will be concretely illustrated by way of the following examples.

Example 1: Production of (-)-(2S,3S)-3-Methoxycarbonyl-1,1-bis(methylthio)-2-(oxazolidin-2-one-3-yl)carbonylcyclobutane

Example 1-1

In an atmosphere of argon gas, 80 ml of 1,3,5-trimethylbenzene (TMB) was added to dichloroisopropoxytitanium (905 mg, 3.8 mmol) and (2S,3S)-2,3-O-(1-phenylethylidene)-1,1,4,4-tetraphenyl-1,2,3,4-butanetetraol (Compound A) 2.22 g, 4.2 mmol), and the mixture was stirred at room temperature for 30 minutes. Then, powdery Molecular Sieves

4A (3.2 g) was added to the resulting solution and stirred for a while, after which 3-[(E)-3-(methoxycarbonyl)-propenoyl]-oxazolidin-2-one (3.98 g, 20 mmol) was added, and the resulting suspension was cooled to -15°C. Then, 1,1-bis(methylthio)ethylene (3.61 g, 30 mmol) dissolved in 20 ml of TMB solution was slowly added, and temperature of the whole mixture was elevated from that temperature to 0°C over a period of 3 hours with stirring. A saturated aqueous solution of sodium hydrogen carbonate was added to the reaction mixture, the inorganic matter was filtered off with Celite, and the organic matter was extracted with ethyl acetate. The extract solution was washed with saturated aqueous solution of sodium chloride and dried over anhydrous sodium sulfate, and the solvent was distilled off under reduced pressure. The residue was purified by silica gel column chromatography (ethyl acetate : hexane = 1 : 2, v/v) to obtain (-)-(2S,3S)-3-methoxycarbonyl-1,1-bis(methylthio)-2-(oxazolidin-2-one-3-yl)-carbonylcyclobutane (3.94 g, 62%). This compound has an optical purity of 86% ee (cf. Example 8).

NMR (500 MHzFT, $CDCl_3$) δ:

2.01(3H, s),
2.10(3H, s),
2.54(1H, dd, J=9.9, 12.3Hz),
2.70(1H, dd, J=7.9, 12.3Hz),
3.86(1H, dt, Jd=9.9Hz, Jt=7.9Hz),
3.71(3H, s),
4.02(1H, ddd, J=6.4, 8.9, 11.0Hz),
4.12(1H, ddd, J=7.6, 9.3, 11.0Hz),
4.39-4.47(2H, m),
5.01(1H, d, J=7.9Hz).

IR (neat) $cm^{-1}$: 1780, 1730, 1690.

Example 1-2

In such a manner as in Example 1-1, 3-[(E)-3-(methoxycarbonyl)-propenoyl]-oxazolidin-2-one (3.98 g, 20 mmol), 1,1-bis(methylthio)ethylene (3.61 g, 30 mmol), dichlorodiisopropoxytitanium (474 mg, 2.0 mmol), (2S,3S)-2,3-O-(1-phenylethylidene)-1,1,4,4-tetraphenyl-1,2,3,4-butanetetraol (Compound A) 1.16 g, 2.2 mmol) and powdery Molecular Sieves 4A (4 g) were reacted in a solvent mixture consisting of toluene (160 ml) and hexane (120 ml) at 0°C for 40 minutes to form (-)-(2S,3S)-3-methoxycarbonyl-1,1-bis(methylthio)-2-(oxazolidin-2-one-3-yl)-carbonylcyclobutane (6.13 g, 96%) ($[\alpha]_D$ = -10.4° (c 1.34, $CH_2Cl_2$)). It was recrystallized from methylene chloride-isopropyl ether system to give a compound ($[\alpha]_D$ = -11.1° (c 1.15, $CH_2Cl_2$)) in a yield of 5.30 g (83%). Optical purity of this compound was 98% ee or above, as determined by the method mentioned in Example 8.

Example 1-3

In such a manner as in Example 1-1, 3-[(E)-3-(methoxycarbonyl)-propenoyl]-oxazolidin-2-one (19.9 g, 100 mmol), 1,1-bis(methylthio)ethylene (15.03 g, 125 mmol), dichlorodiisopropoxytitanium (1.18 g, 5.0 mmol), (2S,3S)-2,3-O-(1-phenylethylidene)-1,1,4,4-tetraphenyl-1,2,3,4-butanetetraol (Compound A) (2.91 g, 5.5 mmol) and powdery Molecular Sieves 4A (20 g) were reacted at 0°C for 5 hours in a solvent mixture consisting of toluene (800 ml) and hexane (600 ml) to obtain (-)-(2S,3S)-3-methoxycarbonyl-1,1-bis(methylthio)-2-(oxazolidin-2-one-3-yl)carbonyl cyclobutane (26.69 g, 84%) ($[\alpha]_D$ = -10.5° (c 1.00, $CH_2Cl_2$)).

Example 2: Production of (+)-(2R,3R)-3-methoxycarbonyl-1,1-bis(methylthio)-2-(oxazolidin-2-one-3-yl)carbonylcyclobutane

In an atmosphere of argon gas, dichloroisopropoxytitanium (125 mg, 0.53 mmol), (2R,3R)-2,3-O-(1-phenylethylidene)-1,1,4,4-tetraphenyl-1,2,3,4-butanetetraol (Compound B) (305 mg, 0.58 mmol) and toluene (5 ml) were mixed together and stirred at room temperature for one hour. A part (0.5 ml, 00.053 mmol) of the resulting solution was taken out and added to powdery Molecular Sieves 4A (100 mg), and toluene (1.5 ml) was added thereto. To the resulting suspension were added 3-[(E)-3-(methoxycarbonyl)-propenoyl]-oxazolidin-2-one (107 mg, 0.534 mmol) and petroleum ether (boiling point ca. 80°C) (PE) (2 ml), and the resulting mixture was cooled to 0°C. To this suspension, a solution of 1,1-bis(methylthio)-ethylene (115 mg, 0.956 mmol) in PE (1.5 ml) was slowly added, and the resulting mixture was stirred at 0°C for 3 hours. Then, 0.2 M phosphate buffer (pH 7) was added to the reaction mixture and the inorganic matter was filtered off with Celite, after which the organic matter was extracted with ethyl acetate. The extract solution was

washed with saturated aqueous solution of sodium chloride and dried over anhydrous sodium sulfate, after which the solvent was distilled off under reduced pressure. The residue was purified by fractionating silica gel thin layer chromatography (ethyl acetate : hexane = 1 : 1, v/v) to give (+)-(2R,3R)-3-methoxycarbonyl-1,1-bis(methylthio)-2-(oxazolidin-2-one-3-yl)-carbonylcyclobutane (162 mg, 95%). This product had an optical purity of 98% ee (cf. Example 9).

NMR and IR of this product were identical with those of the compound of Example 1.

Example 3: Production of (+)-(2R,3R)-3-Methoxycarbonyl-1,1-bis(methylthio)-2-(oxazolidin-2-one-3-yl)-carbonylcyclobutane

The following table illustrates the results of a study on the conditions in the production of this compound (including Example 2). The other conditions and methods of treatment were the same as in Example 1 or 2.

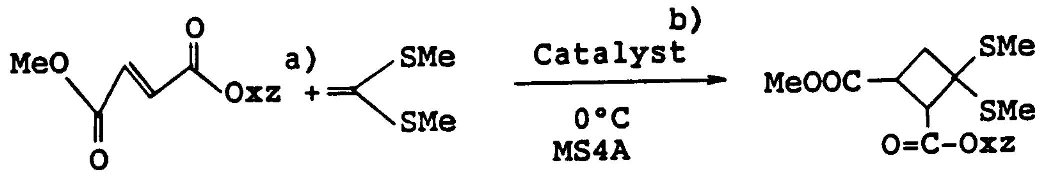


| No. | Solvent | Catalyst (eq) | Time (h) | Yield (%) | Optical purity[c)] (% ee) |
|---|---|---|---|---|---|
| 1 | TMB[d)] | 1.0 | 0.5 | 89 | 93.2 |
| 2 | TMB | 0.1 | 0.5 | 99 | 94.0 |
| 3 | T-PE[e)] | 1.0 | 3 | 78 | 98.3 |
| 4 | T-PE | 0.1 | 3 | 95 | 97.7 |
| 5 | $Et_2O$ | 1.0 | 18 | 45 | - |

a) Oxz is oxazolidin-2-one-3-yl group.

b) A mixture (1.0:1.1) of $TiCl_2(i\text{-}PrO)_2$ and Compound B

c) Optical purity was determined from NMR of bis-MTPA ester of Compound (VII).

d) TMB means 1,3,5-trimethylbenzene.

e) T-PE means toluene-petroleum ether.

Example 4: Production of (+)-1,1-Bis(methylthio)-2-(oxazolidin-2-one-3-yl)-carbonylcyclobutane

The reaction of Example 2 was repeated, except that the 3-[(E)-3-(methoxycarbonyl)propenoyl]-oxazolidin-2-one was replaced with 3-propenoyl-oxazolidin-2-one, to give (+)-1,1-bis(methylthio)-2-(oxazolidin-2-one-3-yl)-carbonylcyclobutane (82%). This product had an optical purity of 88% ee (cf. Example 10).

NMR (500 MHzFT, $CDCl_3$) δ:

2.00 (3H, s),
2.12 (3H, s),
2.26-2.33 (1H, m),
2.34-2.38 (1H, m),
2.39-2.48 (1H, m),
2.52-2.61 (1H, m),
4.00-4.13 (2H, m),
4.41 (2H, t, J=8.0Hz),
4.63 (1H, m).

Example 5: Production of (-)-(2S,3S)-2,3-Bis(methoxycarbonyl)-1,1-bis(methylthio)-cyclobutane

Example 5-1

In an atmosphere of argon gas, 1M-dimethoxymagnesium/methanol (25 ml, 25 mmol) was added to a methanolic solution (25 ml) of (-)-(2S,3S)-3-methoxycarbonyl-1,1-bis(methylthio)-2-(oxazolidin-2-one-3-yl)-carbonylcyclobutane (3.94 g, 12.3 mmol) obtained in Example 1-1 and stirred at room temperature for one hour. The reaction mixture was concentrated under reduced pressure, saturated aqueous solution of ammonium chloride was added to the concentrate, and it was extracted with ether. The ether extract was washed with saturated aqueous solution of sodium chloride, and then the solvent was distilled off under reduced pressure. The residue was purified by silica gel column chromatography (ethyl acetate : hexane = 1 : 9, v/v) to give (-)-(2S,3S)-2,3-bis(methoxycarbonyl)-1,1-bis(methylthio)cyclobutane (2.17 g, 67%). This compound had an optical purity of 86% ee (cf. Example 8).

NMR (500 MHzFT, $CDCl_3$) δ:

2.01 (3H, s),
2.12 (3H, s),
2.47 (1H, dd, J=9.0, 12.2Hz),
2.50 (1H, dd, J=9.4, 12.2Hz),
3.63-3.75 (2H, m),
3.69 (3H, s),
3.72 (3H, s),

IR (neat) $cm^{-1}$: 1730.

Example 5-2

In an atmosphere of argon gas, (-)-(2S,3S)-3-methoxycarbonyl-1,1-bis(methylthio)-2-(oxazolidin-2-one-3-yl)-carbonylcyclobutane (26.0 g, 81.4 mmol) was added to 1M-dimethoxymagnesium/methanol (400 ml, 400 mmol) at 0°C, and the mixture was stirred at that temperature for 15 minutes. Saturated aqueous solution of ammonium chloride was added to the reaction mixture, and it was extracted with ether. After washing the extract solution with saturated aqueous solution of sodium chloride, the solvent was distilled off under reduced pressure. The residue was purified by silica gel column chromatography (ethyl acetate : hexane = 1 : 9, v/v) to give (-)-(2S,3S)-2,3-bis(methoxycarbonyl)-1,1-bis(methylthio)cyclobutane (20.73 g, 96%).

Example 6: production of (+)-(2R,3R)-2,3-Bis(methoxycarbonyl)-1,1-bis(methylthio)-cyclobutane

The procedure of Example 5-2 was repeated, except that (+)-(2R,3R)-3-methoxycarbonyl-1,1-bis(methylthio)-2-(oxazolidin-2-one-3-yl)carbonylcyclobutane produced in Example 2 was used as the starting compound. Thus, (+)-(2R,3R)-2,3-bis(methoxycarbonyl)-1,1-bis(methylthio)cyclobutane was obtained in a yield of 95%. This compound had

an optical purity of 98% ee (cf. Example 9).

NMR and IR of this compound were identical with those of the compound of Example 5.

Example 7: Production of (+)-2-Methoxycarbonyl-1,1-bis(methylthio)cyclobutane

The procedure of Example 6 was repeated, except that the (+)-1,1-bis(methylthio)-2-(oxazolidin-2-on-3-yl)-carbonylcyclobutane produced in Example 4 was used as the starting compound. Thus, (+)-2-methoxycarbonyl-1,1-bis(methylthio)cyclobutane was obtained in a yield of 83%. This compound had an optical purity of 88% ee (cf. Example 10).

NMR (500 MHzFT, $CDCl_3$) δ:

1.97 (3H, s),
2.07 (3H, s),
2.18-2.30 (3H, m),
2.49 (1H, m),
3.37 (1H, m), 3.67 (3H, s).

Example 8: Production of (-)-(2S,3S)-2,3-Bis(hydroxymethyl)-1,1-bis(methylthio)cyclobutane

In an atmosphere of argon gas, an ethereal solution (10 ml) of (-)-(2S,3S)-2,3-bis(methoxycarbonyl)-1,1-bis(methylthio)cyclobutane (1.96 g, 7.4 mmol) produced in Example 5-1 was slowly added to an ethereal suspension of lithium aluminum hydride (562 mg, 14.8 mmol) at 0°C, and the resulting mixture was stirred at 0°C for 2 hours. After adding saturated aqueous solution of sodium sulfate to the reaction mixture to decompose the excessive reductant, anhydrous sodium sulfate was added and stirred for a while. Then, the inorganic matter was filtered off and washed with hot isopropyl alcohol. The filtrate and washings were united and the solvent was distilled off therefrom under reduced pressure. The residue was purified by silica gel column chromatography (ethyl acetate : hexane : methanol = 15 : 20 : 1, v/v/v) to obtain (-)-(2S,3S)-2,3-bis(hydroxymethyl)-1,1-bis(methylthio)cyclobutane (1.48 g, 96%).

NMR (270 MHzFT, $CDCl_3$) δ:

2.02 (1H, dd, J=9.1, 12.1Hz),
2.05 (3H, s),
2.06 (3H, s),
2.28 (1H, dd, J=8.1, 12.1Hz),
2.47-2.68 (2H, m),
3.26 (2H, brs),
3.54 (1H, dd, J=8.8, 10.3Hz),
3.67-3.77 (2H, m),
3.83 (1H, dd, J=5.0, 10.4Hz),

IR(neat) $cm^{-1}$: 3350.

A part of this compound was taken out and converted to bis(R)-TMPA ester in the usual manner by using (R)-α-methoxy-α-trifluoromethyl-phenylacetyl chloride ((R)-MTPACl), dimethylaminopyridine (DMAP)/pyridine (pyr).

In 500 MHz NMR, four signals of methyl group originated from racemic mixture were observed at 1,852, 1,856, 1,912 and 1,970 ppm. Among them, the methyl group signals at 1,852 and 1,912 ppm were greater than the others, from which optical purity was determined as 86% ee.

The title compound (-)-(2S,3S)-2,3-bis(hydroxymethyl)-1,1-bis(methylthio)cyclobutane can be made into a crystal having 100% optical purity by recrystallization from ethyl acetate-hexane system ($[\alpha]_D$ = -32.0° (c 1.03, $CH_2Cl_2$)).

Example 9: Production of (+)-(2R,3R)-2,3-Bis(hydroxymethyl)-1,1-bis(methylthio)-cyclobutane

The procedure of Example 8 was repeated, except that (+)-(2R,3R)-2,3-bis(methoxycarbonyl)-1,1-bis(methylthio)cyclobutane produced in Example 6 was used. Thus, (+)-(2R,3R)-2,3-bis(hydroxymethyl)-1,1-bis(methylthio)cyclobutane was obtained in a yield of 70%.

NMR and IR of this compound were identical with those of the compound of Example 8.

A part of this compound was taken out and converted to (R)-TMPA ester according to usual method ((R)-MTPACl, DMAP/pyr).

NMR of the product was studied according to Example 8. Thus, it was found that the signals of 1,856 and 1,970 ppm were greater than the others, and optical purity was 98% ee.

Apart from the above, a part of the title compound was recrystallized from ethyl acetate-hexane system to give a colorless crystal. By X ray analysis of its single crystal, its absolute steric configuration was determined as (2R,3R), as shown in the title.

Example 10: Production of (+)-2-Hydroxymethyl-1,1-bismethylthio)cyclobutane

The procedure of Example 8 was repeated, except that (+)-2-methoxycarbonyl-1,1-bis(methylthio)cyclobutane obtained in Example 7 was used. Thus, (+)-2-hydroxymethyl-1,1-bis(methylthio)cyclobutane was obtained in a yield of 54%.

NMR (500 MHzFT, $CDCl_3$) δ:

1.92 (1H, m),
2.05 (3H, s),
2.07 (3H, m),
2.12-2.32 (4H, m),
2.68 (1H, m),
3.73 (1H, dd, J=4.8, 11.8Hz),
3.84 (1H, dd, J=7.5, 11.8Hz).

A part of this compound was taken out and converted to (R)-MTPA ester according to usual method ((R)-MTPACl, DMAP/pyr).

In 500 MHz NMR, four methyl group signals of this compound originated from racemic mixture were found at 1,696, 1,927, 1,930 and 1,955 ppm. Among them, the signals of 1,927 and 1,955 ppm were greater than the others, from which optical purity was determined as 88% ee.

Example 11: Production of (+)-(2S,3S)-2,3-Bis(t-butyldiphenylsilyloxymethyl)-1,1-bis(methylthio)cyclobutane

Into methylene chloride (25 ml) were dissolved (-)-(2S,3S)-2,3-bis(hydroxymethyl)-1,1-bis(methylthio)cyclobutane (1.37 g, 6.58 mmol), triethylamine (2.8 ml, 20 mmol), 4-dimethylaminomethylpyridine catalytic amount) and DMF (1 ml). Then, t-butyldiphenylsilyl chloride (4.52 g, 25 mmol) was added to the solution and stirred at room temperature overnight. After concentrating the reaction mixture under reduced pressure, the concentrate was dissolved into ether, washed with saturated aqueous solution of sodium chloride and dried over anhydrous sodium sulfate. After distilling off the solvent from the etheral solution, the residue was purified by silica gel column chromatography (ether : hexane = 1 : 20, v/v) to give (+)-(2S,3S)-2,3-bis((t-butyldiphenylsilyloxymethyl)-1,1-bis(methylthio)cyclobutane (4.50 g, 100%).

NMR (200 MHzFT, $CDCl_3$) δ:

0.99 (9H, s),
1.02 (9H, s),
2.06 (6H, s),
2.09-2.25 (2H, m),
2.85 (1H, m),
3.52-3.73 (3H, m),
3.90 (1H, dd, J=9.0, 10.8Hz),
7.26-7.47 (12H, m),
7.55-7.74 (8H, m),

Example 12: Production of (-)-(2R,3R)-2,3-Bis(t-butyldiphenylsilyloxymethyl)-1,1-bis(methylthio)cyclobutane

t-Butyldiphenylsilyl chloride (292 mg, 1.06 moles) was added to a solution of (+)-(2R,3R)-2,3-bis(hydroxymethyl)-1,1-bis(methylthio)cyclobutane (82 mg, 0.39 mmol), imidazole (106 mg, 1.55 mmol) and 4-dimethylaminomethylpyridine (catalytic amount) in DMF (4 ml), and the resulting mixture was stirred at room temperature overnight. After adding 2M-phosphate buffer (pH 7.0) to the reaction mixture, it was extracted with ethyl acetate. The extract solution was washed with water and then with saturated aqueous solution of sodium chloride and dried over anhydrous sodium sulfate, after which the solvent was distilled off under reduced pressure. The residue was purified by preparing silica gel

TLC (ethyl acetate : hexane = 1 : 10, v/v) to give (-)-(2R,3R)-2,3-bis(t-butyldiphenylsilyloxymethyl)-1,1-bis(methylthio)cyclobutane (266 mg, 100%).

NMR and IR of this compound were identical with those of the compound of Example 11.

Example 13: Production of (+)-(2S,3S)-2,3-Bis(t-butyldiphenylsilyloxymethyl)-1-cyclobutanone

N-Chlorosuccinimide (1.60 g, 12 mmol) and silver nitrate (2.29 g, 13.5 mmol) were dissolved into 80% aqueous solution of acetonitrile (45 ml), to which was rapidly added at 25°C a solution of (+)-(2S,3S)-2,3-bis(t-butyldiphenylsilyloxymethyl)-1,1-bis(methylthio)cyclobutane (2.06 g, 3 mmol) in a mixture consisting of acetonitrile (6 ml) and methylene chloride (1 ml). The resulting mixture was stirred for 10 minutes. After adding 3 ml of saturated aqueous solution of sodium sulfite to the reaction mixture and stirring it for one minute, saturated aqueous solution of sodium hydrogen carbonate (3 ml) was added and stirred for one minute, and then saturated aqueous solution of sodium chloride (3 ml) was added and stirred for one minute. Then, 60 ml of a mixture of methylene chloride and hexane (1 : 1, v/v) was added to this solution and the insoluble matter was filtered off by the use of Hyflo® (manufactured by Johns-Manville Co.). After drying the filtrate over anhydrous sodium sulfate, the solvent was distilled off under reduced pressure, and the residue was purified by silica gel column chromatography (ether : hexane = 1 : 10, v/v) to obtain (+)-(2S,3S)-2,3-bis(t-butyldiphenylsilyloxymethyl)-1-cyclobutanone (1.49 g, 82%).

NMR (270 MHzFT, $CDCl_3$) δ:

1.02 (9H, s),
1.04 (9H, s),
2.74-3.05 (2H, m),
3.29 (1H, m),
3.69 (1H, dd, J=3.7, 10.6Hz),
3.82 (1H, dd, J=4.8, 10.3Hz),
3.88 (1H, dd, J=4.8, 10.3Hz),
3.97 (1H, dd, J=4.0, 10.6Hz),
7.32-7.44 (12H, m),
7.62-7.68 (8H, m),

IR (neat) $cm^{-1}$: 1785.

Example 14: Production of (-)-(2R,3R)-2,3-Bis(t-butyldiphenylsilyloxymethyl)-1-cyclobutanone

The procedure of Example 13 was repeated, except that (-)-(2R,3R)-2,3-bis(t-butyldiphenylsilyloxymethyl)-1,1-bis(methylthio)-cyclobutane was used. Thus, (-)-(2R,3R)-2,3-bis(t-butyldiphenylsilyloxymethyl)-1-cyclobutanone was obtained in a yield of 99%.

NMR and IR of this compound were identical with those of the compound of Example 13.

Example 15: Production of 2,3-Bis(t-butyldiphenylsilyloxymethyl)cyclobutanol

Example 15-1

In an atmosphere of argon gas, lithium tri(t-butoxy)-aluminum hydride (1.27 g, 5.0 mmol) was added to tetrahydrofuran (THF) (10 ml) and cooled to -78°C. To this suspension was added a solution of (+)-(2S,3S)-2,3-bis(t-butyldiphenylsilyloxymethyl)-1-cyclobutanone (1.21 g, 2.0 mmol) in THF, and temperature of the mixture was slowly elevated to room temperature with stirring over a period of several hours. After adding 0.2 M phosphate buffer to the reaction mixture to decompose the excessive reductant, methylene chloride was added and the inorganic matter was filtered off. The filtrate was extracted with methylene chloride, and the methylene chloride layer was dried over anhydrous sodium sulfate, after which the solvent was distilled off. The residue was separated and purified by silica gel column chromatography (ethyl acetate : hexane = 1 : 9 to 1 : 4, v/v) to obtain (+)-(1S,2S,3S)-2,3-bis(t-butyldiphenylsilyloxymethyl)cyclobutanol (0.104 g, 9%):

NMR (200 MHzFT, $CDCl_3$) δ:

1.00 (9H, s),
1.07 (9H, s),

1.97-2.25 (2H, m),
2.32 (1H, m),
2.48 (1H, m),
3.17 (1H, d, J=7.3Hz),
3.56 (2H, d, J=5.8Hz),
3.88 (1H, dd, J=5.6, 11.4Hz),
3.98 (1H, dd, J=4.0, 11.4Hz),
4.46 (1H, m),
7.26-7.48 (12H, m),
7.54-7.72 (8H, m),

and (+)-(1R,2S,3S)-2,3-bis(t-butyldiphenylsilyloxymethyl)cyclobutanol (1.068 g, 88%):

NMR (200MHzFT, $CDCl_3$) $\delta$:

1.03 (9H, s),
1.04 (9H, s),
1.60-1.73 (2H, m),
1.92 (1H, m),
2.10-2.37 (2H, m),
3.52-3.70 (3H, m),
3.77 (1H, dd, J=4.5, 10.4Hz),
4.03 (1H, m),
7.27-7.46 (12H, m),
7.56-7.69 (8H, m).

Example 15-2

In an atmosphere of argon gas, 1M-diisobutylaluminum hydride/toluene (8.2 ml, 8.2 mmol) was slowly added at -78°C to a solution of (+)-(2S,3S)-2,3-bis(t-butyldiphenylsilyloxymethyl)-1-cyclobutanone (4.12 g, 6.8 mmol) in toluene (70 ml), and the mixture was stirred at that temperature for 10 minutes. After adding 0.2 M phosphate buffer (pH 7) to the reaction mixture and stirring it for a while, an excessive amount of methylene chloride was added and the inorganic matter was filtered off. The filtrate was extracted with methylene chloride and dried over anhydrous sodium sulfate, and then the solvent was distilled off. The residue was purified by silica gel column chromatography (ethyl acetate: hexane = 1 : 9 to 1 : 4, v/v) to give (+)-(1S,2S,3S)-2,3-bis(t-butyldiphenylsilyloxymethyl)cyclobutanol (3.38 g, 82%) and (+)-(1R,2S,3S)-2,3-bis(t-butyldiphenylsilyloxymethyl)cyclobutanol (0.69 g, 17%).

Example 15-3: Transformation from (+)-(1R,2S,3S)-2,3-Bis(t-butyldiphenylsilyloxymethyl)-cyclobutanol to (+)-(1S,2S,3S)-2,3-Bis(t-butyldiphenylsilyloxymethyl)cyclobutanol

Step 1

In an atmosphere of argon gas, diethyl azodicarboxylate (217 microliters, 1.38 mmol) was added to a solution of (+)-(1R,2S,3S)-2,3-bis(t-butyldiphenylsilyloxymethyl)cyclobutanol (700 mg, 1.15 mmol), benzoic acid (167 mg, 1.37 mmol) and triphenylphosphine (362 mg, 1.38 mmol) in benzene (10 ml), and the resulting mixture was stirred at room temperature overnight. After distilling off volatile substances from the reaction mixture, the residue was purified by silica gel column chromatography (ether : hexane = 1 : 10, v/v) to give (+)-(1S,2S,3S)-1-benzoyl-2,3-bis(t-butyldiphenylsilyloxymethyl)cyclobutane (791 mg, 97%).

NMR (200 MHzFT, $CDCl_3$) $\delta$:

0.97 (9H, s),
1.06 (9H, s),
2.23-2.40 (2H, m),
2.45 (1H, m),
2.85 (1H, m),
3.71 (2H, d, J=5.4Hz),
3.83 (1H, dd, J=6.1, 10.5Hz),

3.99 (1H, dd, J=7.3, 10.5Hz),
5.48 (1H, apparent q, J=6.5Hz),
7.21-7.45 (14H, m),
7.49-7.72 (9H, m),
7.98 (2H, d, J=7.1Hz).

Step 2

In an atmosphere of argon gas, 1M-diisobutylaluminum hydride/toluene (2.6 ml, 2.6 mmol) was slowly added at -78°C to a solution of (+)-(1S,2S,3S)-1-benzoyl-2,3-bis(t-butyldiphenylsilyloxymethyl)cyclobutane (790 mg, 1.1 mmol) in toluene (10 ml), and the mixture was stirred at that temperature for 30 minutes. 0.2 M Phosphate buffer (pH 7) was added to the reaction mixture and stirred for a while, after which an excessive amount of methylene chloride was added and the inorganic matter was filtered off. The filtrate was extracted with methylene chloride, the methylene chloride layer was dried over anhydrous sodium sulfate, and the solvent was distilled off therefrom. The residue was separated and purified by silica gel column chromatography (ether : hexane = 1 : 5, v/v) to give (+)-(1S,2S,3S)-2,3-bis(t-butyldiphenylsilyloxymethyl)cyclobutanol (644 mg, 95%).

Example 16: Production of (+)-(1R,2S,3S)-2,3-Bis(t-butyldiphenylsilyloxymethyl)-1-methanesulfonyloxycyclobutane

Methanesulfonyl chloride (0.17 ml, 2.2 mmol) was added at 0°C to a solution of (+)-(1R,2S,3S)-2,3-bis(t-butyldiphenylsilyloxymethyl)cyclobutanol (911 mg, 1.5 mmol) and triethylamine (0.6 ml, 4.3 mmol) in methylene chloride, and the mixture was stirred at 0°C for 15 minutes. After adding 0.2 M phosphate buffer to the reaction mixture and extracting it with ether, the extract solution was dried over anhydrous sodium sulfate and the solvent was distilled off. The residue was purified by silica gel column chromatography (ethyl acetate : hexane = 1 : 6, v/v) to give (+)-(1R,2S,3S)-2,3-bis(t-butyldiphenylsilyloxymethyl)-1-methanesulfonyloxycyclobutane (1.034 g, 100%).

NMR (200 MHzFT, $CDCl_3$) δ:

1.04 (9H, m),
1.05 (9H, m),
2.07-2.23 (2H, m),
2.43 (1H, m),
2.64 (2H, m),
2.91 (3H, m),
3.52-3.65 (3H, m),
3.76 (1H, dd, J=4.0, 11.0Hz),
4.97 (1H, m),
7.24-7.48 (12H, m),
7.56-7.70 (8H, m),

Example 17: Production of (-)-9-[(1S,2R,3S)-2,3-Bis(hydroxymethyl)cyclobutane-1-yl]-adenine (comparative example)

Step 1: Production of (+)-9-[(1S,2R,3S)-2,3-bis(t-butyldiphenylsilyloxymethyl)cyclobutane-1-yl]-adenine

To a suspension of adenine (177 mg, 1.3 mmol) in DMF (6.5 ml) was added 60% sodium hydride (52 mg, 1.3 mmol). After stirring the mixture for one hour, a solution of (+)-(1R,2S,3S)-2,3-bis(t-butyldiphenylsilyloxymethyl)-1-methanesulfonyloxycyclobutane (450 mg, 0.65 mmol) in DMF (1.5 ml) was added to the reaction mixture and stirred at 145°C for 6 hours. After cooling, 0.2 M phosphate buffer was added and the mixture was extracted with ethyl acetate. The extract solution was dried over anhydrous sodium sulfate, and then the solvent was dried over anhydrous sodium sulfate, and then the solvent was distilled off. The residue was separated and purified by silica gel column chromatography (methylene chloride : methanol = 50 : 1 to 30 : 1, v/v) to give unreacted (+)-(1R,2S,3S)-2,3-bis(t-butyldiphenylsilyloxymethyl)-1-methanesulfonyloxycyclobutane (100 mg, 22% recovery) and (+)-9-[(1S,2R,3S)-2,3-bis(t-butyldiphenylsilyloxymethyl)cyclobutane-1-yl]-adenine (203 mg, 43%).

NMR (400 MHzFT, $CD_3OD$) δ:

0.75 (9H, s),
1.03 (9H, s),

2.42-2.54 (2H, m),
2.92-3.08 (2H, m),
3.53 (1H, dd, J=4.0, 11.0Hz),
3.68 (1H, dd, J=8.8, 11,0Hz),
3.73-3.85 (2H, m),
5.23 (1H, m),
7.20-7.46 (16H, m),
7.60-7.72 (4H, m),
8.18 (1H, s),

Step 2: Production of (-)-9-[(1S,2R,3S)-2,3-bis(hydroxymethyl)cyclobutane-1-yl]-adenine

To a methanolic solution (1 ml) of (+)-9-[(1S,2R,3S)-2,3-bis(t-butyldiphenylsilyloxymethyl)cyclobutane-1-yl]-adenine (203 mg, 0.28 mmol) obtained in Step 1 was added 4N-hydrochloric acid/dioxane (0.15 ml, 0.6 mmol). After stirring the mixture at room temperature overnight, the solvent was distilled off from the reaction mixture under reduced pressure, and water was added. After removing ether-soluble substances, it was neutralized with 0.1 N sodium hydroxide solution and the solvent was distilled off. The crude product thus obtained was purified by Sephadex LH-20 column chromatography (methanol : water = 1 : 1, v/v) and then washed with acetone to give (-)-9-[(1S,2S,3S)-2,3-bis(hydroxymethyl)cyclobutane-1-yl]-adenine (26 mg, 37%).

NMR (400 MHzFT, $CD_3OD$) δ:

2.43-2.57 (2H, m),
2.78 (1H, m),
3.03 (1H, m),
3.41 (1H, dd, J=6.2, 11.4Hz),
3.46 (1H, dd, J=7.3, 11.4Hz),
3.74 (2H, d, J=6.2Hz),
5.25 (1H, apparent q, J=8.1Hz),
8.20 (1H, s),
8.37 (1H, s).

UV λmax ($H_2O$) nm:

pH 1,258; pH 7,260; pH 13,260.

Example 18: Production of (-)-9-[(1S,2R,3S)-2,3-Bis(hydroxymethyl)cyclobutane-1-yl]-guanine (comparative example)

Step 1: Production of (-)-2-amino-9-[(1S,2R,3S)-2,3-bis(t-butyldiphenylsilyloxymethyl)cyclobutane-1-yl]-6-(2-methoxyethoxy)-purine

Lithium hydride (10 mg, 1.3 mmol) was added to a suspension of 2-amino-6-(2-methoxyethoxy)-purine (274 mg, 1.3 mmol) in DMF (6.5 ml), and the mixture was stirred for one hour. Then, a solution of (+)-(1R,2S,3S)-2,3-bis(t-butyldiphenylsilyloxymethyl)-1-methanesulfonyloxycyclobutane (450 mg, 0.65 mmol) in DMF (1.5 ml) was added to the reaction mixture, and stirred at 145°C for 6 hours. After cooling, 0.2 M phosphate buffer and ethyl acetate were added, the insoluble matter was filtered off, and then the filtrate was extracted with ethyl acetate. The extract solution was dried over anhydrous sodium sulfate, and the solvent was distilled off. The residue was purified by silica gel column chromatography (ethyl acetate : hexane = 5 : 1 to 3 : 1, v/v) to give unreacted (+)-(1R,2S,3S)-2,3-bis(t-butyldiphenylsilyloxymethyl)-1-methanesulfonyloxycyclobutane (157 mg, 35% recovery) and (-)-2-amino-9-[(1S,2R,3S)-2,3-bis(t-butyldiphenylsilyloxymethyl)cyclobutane-1-yl]-6-(2-methoxyethoxy)purine (160 mg, 31%).

NMR (400 MHzFT, $CD_3OD$) δ:

0.79 (9H, s),
1.03 (9H, s),
2.38-2.40 (2H, m),
2.92 (1H, m),
2.98 (1H, m),

3.54 (1H, dd, J=4.0, 11.0Hz),
3.68 (1H, dd, J=8.8, 11.0Hz),
3.72-3.85 (4H, m),
4.56-4.67 (2H, m),
5.18 (1H, apparent q, J=8.0Hz),
7.23-7.47 (16H, m),
7.50-7.60 (4H, m),
8.07 (1H, s).

Step 2: Production of (-)-9-[(1S,2R,3S)-2,3-bis(hydroxymethyl)cyclobutane-1-yl]-guanine

2 N hydrochloric acid (1 ml) was added to (-)-2-amino-9-[(1S,2R,3S)-2,3-bis(t-butyldiphenylsilyloxymethyl)cyclobutane-1-yl]-6-(2-methoxyethoxy)-purine (140 mg, 0.17 mmol) obtained in Step 1, and the mixture was heated under reflux for one hour. After distilling off the solvent from the reaction mixture under reduced pressure, water was added and the ether-soluble substances were removed. Then, it was neutralized with 0.1 N sodium hydroxide solution and the solvent was distilled off. The residue was purified by HP-20 column chromatography (water : methanol = 1 : 0 to 1 : 3, v/v) and then washed with acetone to give (-)-9-[(1S,2R,3S)-2,3-bis(hydroxymethyl)cyclobutane-1-yl]-guanine (15 mg, 32%).

NMR (400 MHzFT, $CD_3OD$) δ:

2.36-2.55 (2H, m),
2.72 (1H, m),
2.90 (1H, m),
3.45 (2H, d, J=7.0Hz),
3.70 (2H, d, J=6.2Hz),
5.05 (1H, apparent q, J=8.1Hz),
7.94 (1H, s),

UV λmax ($H_2O$) nm:

pH 1,253,279(sh); pH 7,252,272(sh);
pH 13,257(sh), 267.

Example 19: Production of (+)-(1S,2S,3S)-2,3-Bis(t-butyldiphenylsilyloxymethyl)-1-methanesulfonyloxycyclobutane

The treatment of Example 16 was repeated, except that (+)-(1S,2S,3S)-2,3-bis(t-butyldiphenylsilyloxymethyl)cyclobutanol was used. Thus, (+)-(1S,2S,3S)-2,3-bis(t-butyldiphenylsilyloxymethyl)-methanesulfonyloxycyclobutane was obtained in a quantitative yield.

NMR (200 MHzFT, $CDCl_3$) δ:

1.04 (9H, s),
1.06 (9H, s),
2.25-2.53 (3H, m),
2.78 (1H, m),
2.87 (3H, s),
3.65 (2H, d, J=4.0Hz),
3.85 (1H, dd, J=6.3, 10.5Hz),
3.93 (1H, dd, J=6.5, 10.5Hz),
5.25 (1H, m),
7.32-7.50 (12H, m),
7.60-7.75 (8H, m).

By recrystallizing this compound from etherhexane system, a crystalline product ($[\alpha]_D$ = +12.0° (C 1.01, $CH_2Cl_2$)) (optical purity 100%) could be obtained.

Example 20: Production of (-)-9-[(1R,2R,3S)-2,3-Bis(hydroxymethyl)cyclobutane-1-yl]-adenine (Compound 1)

Step 1: Production of (+)-9-[(1R,2R,3S)-2,3-bis(t-butyldiphenylsilyloxymethyl)cyclobutane-1-yl]adenine

To a suspension of adenine (100 mg, 0.74 mmol) in DMF (4 ml) was added 60% sodium hydride (30 mg, 0.75 mmol). After stirring the mixture for one hour, a solution of (+)-(1S,2S,3S)-2,3-bis(t-butyldiphenylsilyloxymethyl)-1-methanesulfonyloxycyclobutane (254 mg, 0.37 mmol) in DMF (1.5 ml) was added to the reaction mixture and stirred at 145°C for 6 hours. After cooling, 0.2 M phosphate buffer was added and the mixture was extracted with ethyl acetate. The extract solution was dried over anhydrous sodium sulfate and the solvent was distilled off. The residue was purified by silica gel column chromatography (methylene chloride : methanol = 30 : 1, v/v) to give (+)-9-[(1R,2R,3S)-2,3-bis(t-butyldiphenylsilyloxymethyl)cyclobutane-1-yl]-adenine (126 mg, 47%).

NMR (200 MHzFT, $CDCl_3$) δ

0.98 (9H, s),
1.06 (9H, s),
2.30-2.65 (3H, m),
3.07 (1H, m),
3.62-3.84 (4H, m),
4.81 (1H, m),
5.61 (2H, brs),
7.20-7.52 (12H, m),
7.52-7.75 (8H, m),
7.85 (1H, s),
8.33 (1H, s).

Step 2: Production of (-)-9-[(1R,2R,3S)-2,3-bis(hydroxymethyl)cyclobutane-1-yl]-adenine (Compound 1)

To methanolic solution (2 ml) of (+)-9-[(1R,2R,3S)-2,3-bis(t-butyldiphenylsilyloxymethyl)cyclobutane-1-yl]-adenine (118 mg, 0.16 mmol) obtained in Step 1 was added 4 N-hydrochloric acid/dioxane (0.17 ml, 0.65 mmol), and the mixture was stirred at room temperature overnight. After distilling off the solvent from the reaction mixture under reduced pressure, water was added and ether-soluble substances were removed. Then, it was neutralized with 0.1 N aqueous solution of sodium hydroxide and the solvent was distilled off. The crude product thus obtained was purified by Sephadex HP-20 column chromatography (water : methanol = 1 : 0 to 1 : 1, v/v), and thereafter by silica gel column chromatography (methylene chloride : ethanol = 5 : 1, v/v) and further thereafter by Sephadex LH-20 column chromatograhy (methanol) to give (-)-9-[(1R,2R,3S)-2,3-bis(hydroxymethyl)cyclobutane-1-yl]-adenine (Compound 1) (37 mg, 91%).

NMR (200 MHzFT, $CD_3OD$) δ:

2.24 (1H, m),
2.37 (1H, apparent q, J=9.5Hz),
2.62 (1H, m),
2.88 (1H, m),
3.65-3.74 (4H, m),
4.71 (1H, apparent q, J=8.5Hz),
8.20 (1H, s),
8.26 (1H, s),

UV λmax ($H_2O$) nm:

pH 1,259; pH 7,259; pH 13,259.

HRMS (FAB)

Calcd for $[C_{11}H_{15}N_5O_2+H]^+$ ; 250, 1304
Found: 250, 1305.

By recrystallizing this compound from ether-methanol system, a crystalline product ($[\alpha]_D$ = -44.7° (c 0.98, pyridine)

(optical purity: 98% or above) was obtained.

By repeating the procedure of this example with the corresponding bases, Compounds No. 7, No. 8, No. 9 and No. 10 were also obtained. The physicochemical properties of No. 7 and No. 9 are shown as follows:

No. 7

NMR (200 MHzFT, $CD_3OD$) δ:

2.30-2.46 (2H, m),
2.38 (1H, m),
2.57 (1H, m),
3.51-3.71 (4H, m),
4.54 (1H, m),
5.69 (1H, d, J=8.0Hz),
7.80 (1H, d, J=8.0Hz),

UV λmax ($H_2O$) nm:

pH1,268; pH7,268; pH13,267.

No. 9

NMR (200 MHzFT, $CD_3OD$) δ:

1.78-2.14 (2H, m),
2.20-2.54 (2H, m),
3.50-3.76 (4H, m),
4.50 (1H, m),
5.91 (1H, d, J=7.4Hz),
7.78 (1H, d, J=7.4Hz),

UV λmax ($H_2O$) nm:

pH1,284; pH7,273; pH13,274.

Example 21: Production of (+)-9-[(1R,2R,3S)-Bis(hydroxymethyl)cyclobutane-1-yl]-guanine (Compound 2)

Step 1: Production of (+)-2-amino-9-[(1R,2R,3S)-2,3-bis(t-butyldiphenylsilyloxymethyl)cyclobutane-1-yl]-6-(2-methoxyethoxy)-purine

Lithium hydride (6 mg, 0.75 mmol) was added to a suspension of 2-amino-6-(2-methoxyethoxy)-purine (155 mg, 0.74 mmol) in DMF (4 ml), and the mixture was stirred for one hour. Then, a solution of (+)-(1S,2S,3S)-2,3-bis(t-butyldiphenylsilyloxymethyl)-1-methanesulfonyloxycyclobutane (450 mg, 0.65 mmol) in DMF (1.5 ml) was added to the reaction mixture and stirred at 145°C for 6 hours. After cooling, 0.2 M phosphate buffer and ethyl acetate were added and the insoluble matter was filtered off, and the filtrate was extracted with ethyl acetate. After drying the extract solution over anhydrous sodium sulfate, the solvent was distilled off. The residue was purified by silica gel column chromatography (methylene chloride : ethyl acetate = 10 : 1, v/v) to give (+)-2-amino-9-[(1R,2R,3S)-2,3-bis(t-butyldiphenylsilyloxymethyl)cyclobutane-1-yl]-6-(2-methoxyethoxy)-purine (88 mg, 30%).

NMR (200 MHzFT, $CDCl_3$) δ

1.01 (9H, s),
1.05 (9H, s),
2.25-2.60 (3H, m),
2.92 (1H, m),
3.44 (3H, s),
3.60-3.94 (6H, m),
4.50-4.90 (5H, m),

7.15-7.55 (12H, m),
7.55-7.80 (8H, m),
7.67 (1H, s).

When DMSO and HMPA were used as the solvent of this reaction, the reaction progressed at 75°C and 100°C, respectively, to give results similar to the results in case of DMF.

Step 2: Production of (+)-9-[(1R,2R,3S)-2,3-bis(hydroxymethyl)cyclobutane-1-yl]-guanine (Compound 2)

To methanolic solution (2 ml) of (+)-amino-9-[(1R,2R,3S)-2,3-bis(t-butyldiphenylsilyloxymethyl)cyclobutane-1-yl]-6-(2-methoxyethoxy)-purine (79 mg, 0.10 mmol) obtained in Step 1 was added 4N-hydrochloric acid/dioxane (0.1 ml, 0.4 mmol). After stirring the mixture overnight at room temperature, the solvent was distilled off from the reaction mixture under reduced pressure. Then, water was added, ether-soluble substances were removed, the solvent was distilled off, 2 N-hydrochloric acid (2 ml) was added, and the mixture was heated under reflux for one hour. After neutralizing the reaction mixture with 1 M aqueous solution of sodium hydroxide, it was purified by Sephadex HP-20 column chromatography (water : methanol = 1 : 0 to 1 : 4, v/v) to give (+)-9-[(1R,2R,3S)-2,3-bis(hydroxymethyl)cyclobutane-1-yl]-guanine (Compound 2) (23 mg, 88%).

NMR (200 MHzFT, $CD_3OD$) δ:

2.18 (1H, m),
2.32 (1H, apparent q, J=10.0Hz),
2.50 (1H, m),
2.79 (1H, m),
3.62-3.74 (4H, m),
4.54 (1H, apparent q, J=8.8Hz),
7.89 (1H, s),

UV λmax ($H_2O$) nm:

pH 1,253,277(sh); pH 7,253,268(sh);
pH 13,256(sh), 267.

HRMS (FAB)

Calcd for $[C_{11}H_{15}N_5O_3+H]^+$; 266, 1253
Found: 266, 1251.

By recrystallizing this compound from water, a crystalline product ($[\alpha]_D$ = +26.5° (c 0.99, 0.1N-NaOH) (optical purity: 98% or above) could be obtained.

By repeating the procedure of this example with the corresponding bases, compounds, No. 3, No. 4, No. 5 and No. 6 were obtained similarly.

Example 22: Production of (1R,2R,3S)-1-Amino-2,3-bis(t-butyldiphenylsilyloxymethyl)cyclobutane

Step 1: Production of (1R,2R,3S)-1-azido-2,3-bis(t-butyldiphenylsilyloxymethyl)cyclobutane

In an atmosphere of argon gas, sodium azide (975 mg, 15 mmol) was added to a solution of (+)-(1S,2S,3S)-2,3-bis(t-butyldiphenylsilyloxymethyl)-1-methane sulfonyloxycyclobutane (1.03 g, 1.5 mmol) in DMF (6 ml), and the mixture was stirred at 120°C for 2 hours. After cooling, the reaction mixture was diluted with water and extracted with ether, and the ether extract solution was washed twice with water and then with saturated aqueous solution of sodium chloride. After drying the ethereal solution over anhydrous sodium sulfate, the solvent was distilled off under reduced pressure and the residue was purified by silica gel column chromatography (methylene chloride : hexane = 1 : 4, v/v) to give (1R,2R,3S)-1-azido-2,3-bis(t-butyldiphenylsilyloxymethyl)cyclobutane (992 mg, quantitative yield).

IR (neat) $cm^{-1}$: 2100.
NMR (200 MHzFT, $CDCl_3$) δ:

1.03 (9H, s),
1.05 (9H, s),
1.91 (1H, m),
2.02-2.29 (2H, m),
2.44 (1H, m),
3.51-3.65 (4H, m),
3.71 (1H, dd, J=4.0Hz, 10.8Hz),
7.31-7.44 (12H, m),
7.57-7.67 (8H, m).

Step 2: Production of (1R,2R,3S)-1-amino-2,3-bis(t-butyldiphenylsilyloxymethyl)cyclobutane

The (1R,2R,3S)-1-azido-2,3-bis(t-butyldiphenylsilyloxymethyl)cyclobutane (992 mg) produced in Step 1 was dissolved into ethyl acetate (2 ml). After adding 10% palladium-carbon (100 mg), the solution was stirred in an atmosphere of hydrogen at room temperature overnight. After filtering off the catalyst, the solvent was distilled off under reduced pressure, and the residue was purified by silica gel column chromatography (hexane : ethyl acetate : triethylamine = 20 : 10 : 1, v/v/v) to give (1R,2R,3S)-1-amino-2,3-bis(t-butyldiphenylsilyloxymethyl)cyclobutane (642 mg, 67%).

NMR (200 MHzFT, $CDCl_3$) δ:

1.03 (9H, s),
1.05 (9H, s),
1.42 (1H, m),
1.54 (2H, brs, exchangeable with $D_2O$),
1.86-2.05 (2H, m),
2.24 (1H, m),
3.13 (1H, m),
3.54-3.65 (3H, m),
3.75 (1H, dd, H=4.1, 10.6Hz),
7.27-7.46 (12H, m),
7.58-7.69 (8H, m).

Example 23: Production of 1-[(1R,2R,3S)-2,3-Bis(hydroxymethyl)cyclobutane-1-yl]-5-methyl-2,4(1H,3H)pyrimidindione (Compound 8)

Step 1: Production of N-[[(1R,2R,3S)-2,3-bis(t-butyldiphenylsilyloxymethyl)cyclobutyl]aminocarbonyl]-3-methoxy-2-methylacrylamide

In an atmosphere of argon gas, a suspension of 3-methoxy-2-methylacrylic acid chloride (673 mg, 5 mmol) and silver cyanate (1.50 g, 10 mmol) in anhydrous benzene (10 ml) was heated under reflux for one hour, and then allowed to stand at room temperature. Supernatant (2.6 ml) of this mixture was taken out and added at 0°C to a solution of (1R,2R,3S)-1-amino-2,3-bis(t-butyldiphenylsilyloxymethyl)cyclobutane (603 mg, 0.99 mmol) in anhydrous benzene (4 ml), and the resulting mixture was stirred overnight as it was. Then, the solvent was distilled off from the reaction mixture under reduced pressure, and the residue was purified by silica gel column chromatography (hexane : ether = 1 : 1, v/v) to give crude N-[[(1R,2R,3S)-2,3-bis(t-butyldiphenylsilyloxymethyl)cyclobutyl]aminocarbonyl]-3-methoxy-2-methylacrylamide (592 mg).

Step 2: Production of 1-[(1R,2R,3S)-2,3-bis(hydroxymethyl)cyclobutan-1-yl]-5-methyl-2,4(1H,3H)pyrimidindione (Compound 8)

1M-Tetrabutylammonium fluoride/THF (2.4 ml, 2.4 mmol) was added to a solution of the N-[[(1R,2R,3S)-2,3-bis(t-butyldiphenylsilyloxymethyl)cyclobutyl]aminocarbonyl]-3-methoxy-2-methylacrylamide (590 mg) obtained in Step 1 in methanol (8 ml), and the mixture was stirred overnight at room temperature. After distilling off the solvent from the reaction mixture, water and ether were added and it was extracted with water six times. The water extracts were concentrated under reduced pressure, 1M sulfuric acid (10 ml) was added thereto, and the mixture was heated under reflux for 30 minutes. The reaction mixture was neutralized with sodium hydroxide solution and purified with Sephadex HP-20 (water-30% aqueous methanol) and thereafter with Dowex 50w-X8 (H-form, water). After neutralizing the eluate with sodium hydroxide solution, it was again purified with Sephadex HP-20 (water-30% aqueous methanol) to give 1-[(1R,2R,3S)-2,3-bis(hydroxymethyl)cyclobutane-1-yl]-5-methyl-2,4-(1H,3H)-pyrimidindione (compound 8) (127 mg, 67%).

NMR (200 MHzFt, $CD_3OD$) δ:

1.90 (3H, d, J=1.1Hz),
1.97-2.18 (2H, m),
2.36 (1H, m),
2.58 (1H, m),
3.57-3.70 (4H, m),
4.55 (1H, m),
7.63 (1H, d, J=1.1Hz),

UV λmax ($H_2O$) nm:

pH 1,273; pH 7,273; pH 13,271.

Example 24: Production of 2-Amino-9-[(1R,2R,3S)-2,3-bis(hydroxymethyl)cyclobutane-1-yl]-6-chloropurine (Compound 4)

Step 1: production of 2-amino-9-[(1R,2R,3S)-2,3-bis(acetoxymethyl)cyclobutane-1-yl]-6-chloropurine

Pyridine (0.5 ml) and acetic anhydride (1 ml) were added to a suspension of 9-[(1R,2R,3S)-2,3-bis(hydroxymethyl)cyclobutane-1-yl]-guanine (Compound 11) (265 mg, 1.0 mmol) in anhydrous dimethylformamide (1.5 ml), and the mixture was stirred at 75°C for 30 minutes. After distilling off the solvent under reduced pressure, the residual solvent was eliminated by azotropically distilling it together with toluene several times, after which the residue was dried. Thus, crude 9-[(1R,2R,3S)-2,3-bis(acetoxymethyl)cyclobutane-1-yl]-guanine (350 mg) was obtained.

The crude 9-[(1R,2R,3S)-2,3-bis(acetoxymethyl)cyclobutane-1-yl]-guanine (350 mg) thus obtained was dissolved into anhydrous acetonitrile (2 ml) together with tetraethylammonium chloride (331 mg, 2.0 mmol), to which were added N,N-dimethylaniline (130 microliters, 1.0 mmol) and phosphorus oxychloride (0.57 ml, 6.0 mmol). The mixture was heated under reflux at 100°C for 10 minutes. After distilling off volatile substances from the reaction mixture under reduced pressure, crushed ice and methylene chloride were added to the residue and stirred at 0°C for a while, and then it was neutralized with saturated aqueous solution of sodium hydrogen carbonate. After stirring the mixture at 0°C for a while, it was extracted with methylene chloride. The methylene chloride extract solution was dried over anhydrous sodium sulfate, and then the solvent was distilled off. The residue was purified by silica gel column chromatography (methylene chloride : ethyl acetate = 1 : 1, v/v) to give 2-amino-9-[(1R,2R,3S)-2,3-bis(acetoxymethyl)cyclobutane-1-yl]-6-chloropurine (335 mg, 91%).

NMR (200 MHzFT, $CDCl_3$) δ:

2.02 (3H, s),
2.11 (3H, s),
2.18-2.73 (3H, m),
3.09 (1H, m),
4.25 (4H, d, J=5.9Hz),
4.59 (1H, apparent q, J=8.4Hz),
4.65 (2H, br),
7.88 (1H, s).

Step 2: Production of 2-amino-9-[(1R,2R,3S)-2,3-bis(hydroxymethyl)cyclobutane-1-yl]-6-chloropurine (Compound 4)

2-Amino-9-[(1R,2R,3S)-2,3-bis(acetoxymethyl)cyclobutane-1-yl]-6-chloropurine (98 mg, 0.27 mmol) was dissolved into methanol (2 ml), mixed with potassium carbonate (90 mg, 0.65 mmol) and stirred at 0°C for 30 minutes. The reaction mixture was neutralized with 1N-hydrochloric acid and then purified with Sephadex HP-20 (water-60% aqueous methanol) to give 2-amino-9-[(1R,2R,3S)-2,3-bis(hydroxymethyl)cyclobutane-1-yl]-6- chloropurine (Compound 4) (65 mg, 86%).

NMR (200 MHzFT, $CD_3OD$) δ:

2.20 (1H, m),
2.37 (1H, m),
2.57 (1H, m),
2.89 (1H, m),
3.69 (4H, d, J=5.5Hz),
4.64 (1H, apparent q, J=8.8Hz),
8.19 (1H, s).

UV $\lambda$max ($H_2O$) nm:

pH 1,244(sh), 313; pH 7,248(sh), 3.6;
pH 13,248(sh), 305.

Example 25: Production of 2-Amino-9-[(1R,2R,3S)-2,3-bis(hydroxymethyl)cyclobutan-1-yl]-purine (Compound 3)

Step 1: Production of 2-amino-9-[(1R,2R,3S)-2,3-bis(acetoxymethyl)cyclobutane-1-yl]-purine

2-Amino-9-[(1R,2R,3S)-2,3-bis(acetoxymethyl)cyclobutane-1-yl]-6-chloropurine (87 mg, 0.24 mmol) was dissolved into methanol (2 ml). After adding 10% palladium carbon (13 mg), it was stirred overnight at room temperature in an atmosphere of hydrogen gas. After adding a small amount of saturated aqueous solution of potassium hydrogen carbonate to the reaction mixture and filtering off the catalyst, the solvent was distilled off under reduced pressure and the residue was purified by silica gel column chromatography (methylene chloride : methanol = 20 : 1, v/v) to give 2-amino-9-[(1R,2R,3S)-2,3-bis(acetoxymethyl)cyclobutane-1-yl]-purine (56 mg, 71%).

NMR (200 MHzFT, $CDCl_3$) $\delta$:

2.01 (3H, s),
2.12 (3H, s),
2.28-2.71 (3H, m),
3.11 (1H, m),
4.17-4.34 (4H, m),
4.60 (1H, apparent q. J=8.6Hz),
5.11 (2H, brs),
7.80 (1H, s),
8.70 (1H, s).

Step 2: Production of 2-amino-9-[(1R,2R,3S)-2,3-bis(hydroxymethyl)cyclobutane-1-yl]-purine (Compound 3)

2-Amino-9-[(1R,2R,3S)-2,3-bis(acetoxymethyl)cyclobutane-1-yl]-chloropurine (40 mg, 0.12 mmol) was dissolved into methanol (2 ml), mixed with potassium carbonate (40 mg, 0.29 mmol) and stirred at 0°C for 30 minutes. After neutralizing the reaction mixture with 0.1 N hydrochloric acid, it was purified with Sephadex HP-20 (water-50% aqueous methanol) to give 2-amino-9-[(1R,2R,3S)-2,3-bis(hydroxymethyl)cyclobutane-1-yl]purine (Compound 3) (27 mg, 90%).

NMR (200 MHzFT, $CD_3OD$) $\delta$:

2.10-2.66 (3H, m),
2.90 (1H, m),
3.70 (4H, d, J=5.6Hz),
4.65 (1H, apparent q, J=8.7Hz),
8.20 (1H, s),
8.54 (1H, s).

UV $\lambda$max ($H_2O$) nm:

pH 1,252(sh), 312; pH 7,244(sh), 304;
pH 13,244(sh), 304.

Example 26: Production of 2,6-Diamino-9-[(1R,2R,3S)-2,3-bis(hydroxymethyl)cyclobutane-1-yl]purine (Compound 5)

2-Amino-9-[(1R,2R,3S)-2,3-bis(acetoxymethyl)cyclobutane-1-yl]-chloropurine (87 mg, 0.24 mmol) was dissolved into methanol (2 ml) and cooled to -78°C. The solution thus obtained was saturated with liquid ammonia, sealed into an ampoule and heated at 100°C for 12 hours. After distilling off volatile substances from the reaction mixture under reduced pressure, it was dissolved into water, neutralized with 0.1 N sodium hydroxide solution and purified with Sephadex HP-20 (water-40% aqueous methanol) to give 2,6-diamino-9-[(1R,2R,3S)-2,3-bis(hydroxymethyl)cyclobutane-1-yl]-purine (Compound 5) (41 mg, 82%).

NMR (200 MHzFT, $CD_3OD$) δ:

2.19 (1H, m),
2.31 (1H, m),
2.54 (1H, m),
2.75 (1H, m),
3.61-3.74 (4H, m),
4.49 (1H, apparent q, J=8.6Hz),
7.90 (1H, s).

UV λmax ($H_2O$) nm:

pH 1,253,291; pH 7,255,280; pH 13,255,280.

Example 27: Production of 9-[(1R,2R,3S)-2,3-Bis(hydroxymethyl)cyclobutane-1-yl]-hypoxanthine (Compound 6)

9-[(1R,2R,3S)-2,3-Bis(hydroxymethyl)-cyclobutane-1-yl]-adenine (Compound 8) (25 mg, 0.10 mmol) was dissolved into water (2.5 ml), mixed with sodium nitrate (138 mg, 2.0 mmol) and cooled to 0°C. After adding acetic acid (0.13 ml), the mixture was stirred at room temperature for one day. The reaction mixture was neutralized with 1 N sodium hydroxide solution and then purified with Sephadex HP-20 (water-40% aqueous methanol) to give 9-[(1R,2R,3S)-2,3-bis(hydroxymethyl)cyclobutane-1-yl]-hypoxanthine (Compound 6) (23 mg, 90%).

NMR (200 MHzFT, $CD_3OD$) δ:

2.25 (1H, m),
2.38 (1H, m),
2.58 (1H, m),
2.90 (1H, m),
3.66-3.74 (4H, m),
4.76 (1H, apparent q, J=8.5Hz),
8.04 (1H, s),
8.20 (1H, s).

UV λmax ($H_2O$) nm:

pH 1,250; pH 7,250; pH 13,254.

## Claims

### Claims for the following Contracting States : BE, DE, FR, GB, IT, NL, SE

1. (1R,2R,3S)cyclobutane derivative represented by the following general formula (IV):

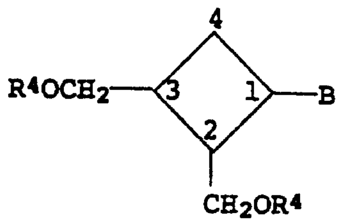

(IV)

wherein $R_4$ represents hydrogen atom and B represents a pyrimidine base represented by

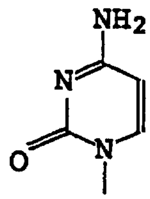

or

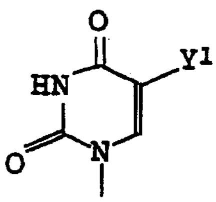

or a purine base represented by

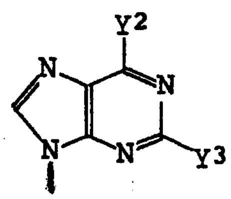

or

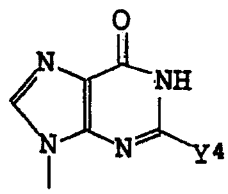

wherein $Y^1$ represents hydrogen atom or $C_{1-4}$ lower alkyl group; $Y^2$ represents hydrogen atom, halogen atom or amino group; $Y^3$ represents hydrogen atom or amino group; and $Y^4$ represents hydrogen atom or amino group.

**2.** (1R,2R,3S) cyclobutane derivative according to Claim 1, wherein B is the purine base.

**3.** (1R,2R,3S) cyclobutane derivative according to Claim 1, wherein B is adenine.

**4.** (1R,2R,3S) cyclobutane derivative according to Claim 1, wherein B is guanine.

**5.** 9-[(1R,2R,3S)-2,3-Bis(hydroxymethyl)cyclobutane-1-yl]-adenine.

**6.** 9-[(1R,2R,3S)-2,3-Bis(hydroxymethyl)cyclobutane-1-yl]-guanine.

**7.** 9-[(1R,2R,3S)-2,3-Bis(hydroxymethyl)cyclobutane-1-yl]-hypoxanthine.

**8.** 2,6-Diamino-9-[(1R,2R,3S)-2,3-bis(hydroxymethyl)cyclobutane-1-yl]purine.

**9.** 2-Amino-9-[(1R,2R,3S)-2,3-bis(hydroxymethyl)cyclobutane-1-yl]-6-chloropurine.

**10.** 2-Amino-9-[(1R,2R,3S)-2,3-bis(hydroxymethyl)cyclobutane-1-yl]purine.

**11.** 2-Amino-9-[(1R,2R,3S)-2,3-bis(acetoxymethyl)cyclobutane-1-yl]purine.

12. An antiviral agent comprising, as its active ingredient, (1R,2R,3S) cyclobutane derivative as claimed in Claim 1 or 2.

13. An antiviral agent comprising, as its active ingredient, 9-[(1R,2R,3S)-2,3-Bis(hydroxymethyl)cyclobutane-1-yl]-adenine.

14. An antiviral agent comprising, as its active ingredient, 9-[(1R,2R,3S)-2,3-Bis(hydroxymethyl)cyclobutane-1-yl]-guanine.

15. The antiviral agent according to Claim 12, 13 or 14, wherein said virus is Herpes Simplex Virus, Cytomegalovirus, B Hepatitis Virus or Immunodeficiency Virus.

16. (1R,2R,3S) cyclobutane derivative as claimed in Claim 1 or 2 for use as an antiviral agent.

17. 9-[(1R,2R,3S)-2,3-Bis(hydroxymethyl)cyclobutane-1-yl]-adenine for use as an antiviral agent.

18. 9-[(1R,2R,3S)-2,3-Bis(hydroxymethyl)cyclobutane-1-yl]-guanine for use as an antiviral agent.

19. The use of 9-[(1R,2R,3S)-2,3-Bis(hydroxymethyl)cyclobutane-1-yl]-adenine for the manufacture of an antiviral agent against B Hepatitis Virus.

20. The use of 9-[(1R,2R,3S)-2,3-Bis(hydroxymethyl)cyclobutane-1-yl]-adenine for the manufacture of an antiviral agent against Human Immunodeficiency Virus.

21. The use of 9-[(1R,2R,3S)-2,3-Bis(hydroxymethyl) cyclobutane-1-yl)-guanine for the manufacture of an antiviral agent against Herpes Simplex Virus.

22. The use of 9-[(1R,2R,3S)-2,3-Bis(hydroxymethyl)cyclobutane-1-yl]-guanine for the manufacture of an antiviral agent against Cytomegalo virus.

23. The use of 9-[(1R,2R,3S)-2,3-Bis(hydroxymethyl)cyclobutane-1-yl]-guanine for the manufacture of an antiviral agent against B Hepatitis Virus.

24. The use of 9-[(1R,2R,3S)-2,3-Bis(hydroxymethyl)cyclobutane-1-yl]-guanine for the manufacture of an antiviral agent against Human Immunodeficiency Virus.

25. (1R,2R,3S)cyclobutane derivative represented by the following general formula (IV):

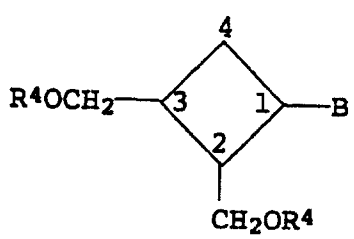

(IV)

wherein $R_4$ represents a conventionally used protecting group and B represents a pyrimidine base represented by

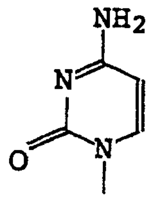

or

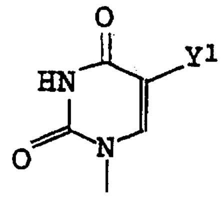

or a purine base represented by

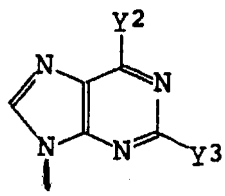

or

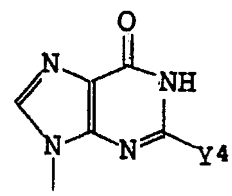

wherein $Y^1$ represents hydrogen atom or $C_{1-4}$ lower alkyl group; $Y^2$ represents hydrogen atom, halogen atom or amino group; $Y^3$ represents hydrogen atom or amino group; and $Y^4$ represents hydrogen atom or amino group.

**26.** (1R,2R,3S) cyclobutane derivative according to Claim 25, wherein the conventionally used protecting group of $R^4$ is an ester type protecting group or an ether type protecting group.

**27.** (1R,2R,3S) cyclobutane derivative according to Claim 25, wherein the conventionally used protecting group of $R^4$ is an acetyl group.

**Claims for the following Contracting State : ES**

**1.** A process for producing (1R,2R,3S)cyclobutane derivative represented by the following general formula (Z):

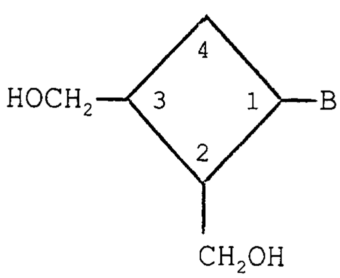

(Z)

wherein B represents a pyrimidine base represented by

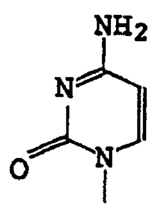

or

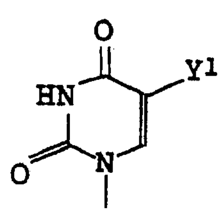

or a purine base represented by

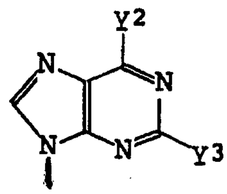

or

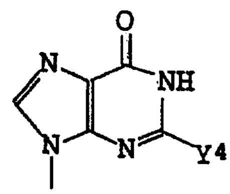

wherein $Y^1$ represents hydrogen atom or $C_{1-4}$ lower alkyl group; $Y^2$ represents hydrogen atom, halogen atom or amino group; $Y^3$ represents hydrogen atom or amino group; and $Y^4$ represents hydrogen atom or amino group, which comprises reacting a compound represented by the following general formula (V) in an optically active form

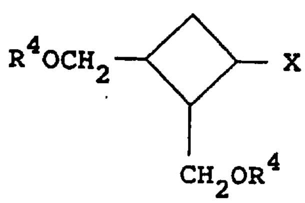

(V)

wherein $R^4$ represents hydrogen atom or conventionally used protecting group and X represents a leaving group, with a corresponding nucleic acid base in a solvent and, when the compound formed by this reaction has a protecting group, eliminating said protecting group.

2. The process according to Claim 1, wherein the conventionally used protecting group of $R^4$ is an ester type protecting group or an ether type protecting group.

3. The process according to Claim 2, wherein said ester type protecting group is acetyl group, benzoyl group, dimethylcarbamoyl group or diphenylcarbamoyl group, and said ether type protecting group is t-butyldimethylsilyl group, t-butyldiphenylsilyl group, methoxymethyl group or benzyl group.

4. The process according to Claim 1, wherein said leaving group is sulfonyloxy group or halogen atom.

5. The process according to Claim 1, wherein B is the purine base.

6. The process according to Claim 1, wherein B is adenine.

7. The process according to Claim 1, wherein B is guanine.

8. The process according to Claim 1, wherein the (1R,2R,3S) cyclobutane derivative is 9-[(1R,2R,3S)-2,3-Bis(hydroxymethyl)cyclobutane-1-yl]-adenine.

9. The process according to Claim 1, wherein the (1R,2R,3S) cyclobutane derivative is 9-[(1R,2R,3S)-2,3-Bis(hydroxymethyl)cyclobutane-1-yl]-guanine.

10. The process according to Claim 1, wherein the (1R,2R,3S) cyclobutane derivative is 9-[(1R,2R,3S)-2,3-Bis(hydroxymethyl)cyclobutane-1-yl]-hypoxanthine.

11. The process according to Claim 1, wherein the (1R,2R,3S) cyclobutane derivative is 2,6-diamino-9-[(1R,2R,3S)-2,3-bis(hydroxymethyl)cyclobutane-1-yl]purine.

12. The process according to Claim 1, wherein the (1R,2R,3S) cyclobutane derivative is 2-amino-9-[(1R,2R,3S)-2,3-bis(hydroxymethyl)cyclobutane-1-yl]-6-chloropurine.

13. The process according to Claim 1, wherein the (1R,2R,3S) cyclobutane derivative is 2-amino-9-[(1R,2R,3S)-2,3-bis(hydroxymethyl)cyclobutane-1-yl]purine.

14. The process according to Claim 1, wherein the (1R,2R,3S) cyclobutane derivative is 2-amino-9-[(1R,2R,3S)-2,3-bis(acetoxymethyl)cyclobutane-1-yl]purine.

15. The process according to Claim 1, wherein the reaction is carried out in a solvent at a temperature ranging from 0°C to the reflux temperature of the solvent.

16. A process for producing (1R,2R,3S) cyclobutane derivative represented by the general formula (Z)

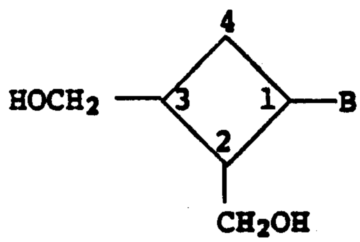

(Z)

wherein B represents a pyrimidine base represented by

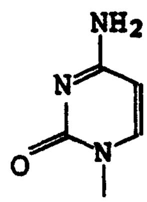

or

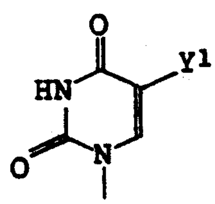

or a purine base represented by

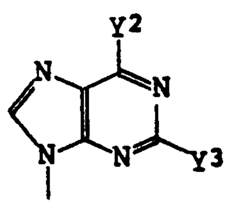

or

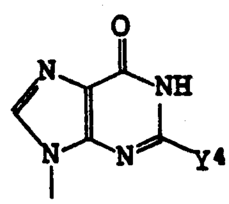

wherein $Y^1$ represents hydrogen atom or $C_{1-4}$ lower alkyl group, $Y^2$ represents hydrogen atom, halogen atom or amino group, $Y^3$ represents hydrogen atom or amino group, and $Y^4$ represents hydrogen atom or amino group, which comprises eliminating a protecting group of (1R,2R,3S) cyclobutane derivative represented by the following general formula (IV):

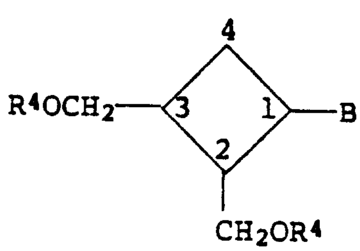

(IV)

wherein $R^4$ represents a conventionally used protecting group and B is as defined above.

**17.** A process for producing (1R,2R,3S)cyclobutane derivative represented by the following general formula (IV):

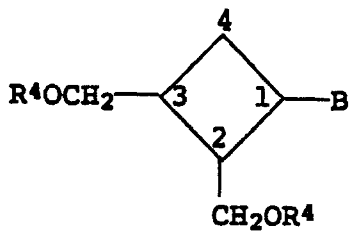

(IV)

wherein $R_4$ represents a conventionally used protecting group and B represents a pyrimidine base represented by

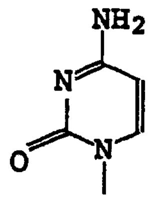

or

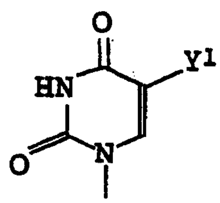

or a purine base represented by

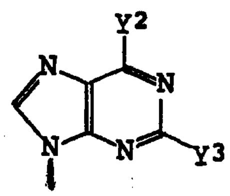

or

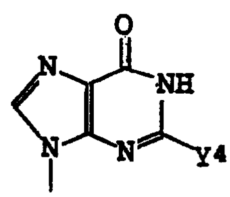

wherein $Y^1$ represents hydrogen atom or $C_{1-4}$ lower alkyl group; $Y^2$ represents hydrogen atom, halogen atom or amino group; $Y^3$ represents hydrogen atom or amino group; and $Y^4$ represents hydrogen atom or amino group, which comprises reacting a compound represented by the following general formula (V) in an optically active form

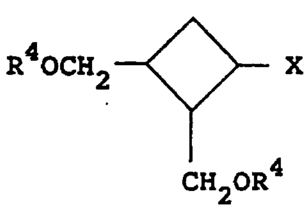

(V)

wherein R4 is as defined above, and X represents a leaving group, with a corresponding nucleic acid base in a solvent and eliminating said protecting group.

### Patentansprüche

### Patentansprüche für folgende Vertragsstaaten : BE, DE, FR, GB, IT, NL, SE

**1.** (1R,2R,3S)Cyclobutan-Derivate, dargestellt durch die folgende Formel (IV):

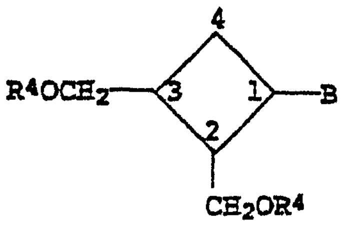

(IV)

worin $R^4$ ein Wasserstoffatom darstellt und B eine Pyrimidinbase, dargestellt durch

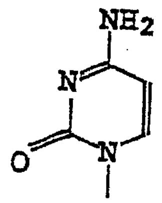

oder

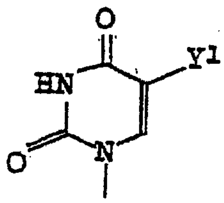

oder eine Purinoase, dargestellt durch

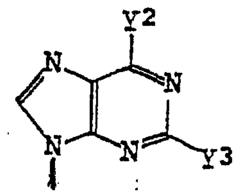

oder

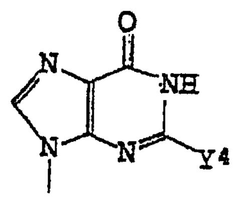

ist, worin $Y^1$ ein Wasserstoffatom oder eine ($C_1$-$C_4$)-Niederalkylgruppe darstellt; $Y^2$ ein Wasserstoffatom, ein Halogenatom oder eine Aminogruppe darstellt; $Y^3$ ein Wasserstoffatom oder eine Aminogruppe darstellt; und $Y^4$ ein Wasserstoffatom oder eine Aminogruppe darstellt.

**2.** (1R,2R,3S)Cyclobutan-Derivate nach Anspruch 1, worin B eine Purinbase ist.

**3.** (1R,2R,3S)Cyclobutan-Derivate nach Anspruch 1, worin B Adenin ist.

**4.** (1R,2R,3S)Cyclobutan-Derivate nach Anspruch 1, worin B Guanin ist.

**5.** 9-[(1R,2R,3S)-2,3-Bis(hydroxymethyl)cyclobutan-1-yl]-adenin.

**6.** 9-[(1R,2R,3S)-2,3-Bis(hydroxymethyl)cyclobutan-1-yl]-guanin.

**7.** 9-[(1R,2R,3S)-2,3-Bis(hydroxymethyl)cyclobutan-1-yl]-hypoxanthin.

**8.** 2,6-Diamino-9-[(1R,2R,3S)-2,3-bis(hydroxymethyl)-cyclobutan-1-yl]-purin.

**9.** 2-Amino-9-[(1R,2R,3S)-2,3-bis(hydroxymethyl)cyclobutan-1-yl]-6-chloropurin.

**10.** 2-Amino-9-[(1R,2R,3S)-2,3-bis(hydroxymethyl)cyclobutan-1-yl]purin.

**11.** 2-Amino-9-[(1R,2R,3S)-2,3-bis(acetoxymethyl)cyclobutan-1-yl]purin.

12. Antivirales Mittel, das als aktiven Bestandteil ein (1R,2R,3S)Cyclobutan-Derivat nach Anspruch 1 oder 2 umfaßt.

13. Antivirales Mittel, das als aktiven Bestandteil 9-[(1R,2R,3S)-2,3-Bis(hydroxymethyl)cyclobutan-1-yl]-adenin umfaßt.

14. Antivirales Mittel, das als aktiven Bestandteil 9-[(1R,2R,3S)-2,3-Bis(hydroxymethyl)cyclobutan-1-yl]-guanin umfaßt.

15. Antivirales Mittel nach Anspruch 12, 13 oder 14, worin der Virus der Herpes-simplex-Virus, der Cytomegalievirus, der Hepatitis B-Virus oder ein Immundefekt-Virus ist.

16. (1R,2R,3S)Cyclobutan-Derivate nach Anspruch 1 oder 2 zur Verwendung als antivirales Mittel.

17. 9-[(1R,2R,3S)-2,3-Bis(hydroxymethyl)cyclobutan-1-yl]-adenin zur Verwendung als antivirales Mittel.

18. 9-[(1R,2R,3S)-2,3-Bis(hydroxymethyl)cyclobutan-1-yl]-guanin zur Verwendung als antivirales Mittel.

19. Verwendung von 9-[(1R,2R,3S)-2,3-Bis(hydroxymethyl)cyclobutan-1-yl]-adenin zur Herstellung eines antiviralen Mittels gegen Hepatitis B-Viren.

20. Verwendung von 9-[(1R,2R,3S)-2,3-Bis(hydroxymethyl)cyclobutan-1-yl]-adenin zur Herstellung eines antiviralen Mittels gegen ein Human-Immundefekt-Virus.

21. Verwendung von 9-[(1R,2R,3S)-2,3-Bis(hydroxymethyl)cyclobutan-1-yl]-guanin zur Herstellung eines antiviralen Mittels gegen das Herpes-simplex-Virus.

22. Verwendung von 9-[(1R,2R,3S)-2,3-Bis(hydroxymethyl)cyclobutan-1-yl]-guanin zur Herstellung eines antiviralen Mittels gegen das Cytomegalievirus.

23. Verwendung von 9-[(1R,2R,3S)-2,3-Bis(hydroxymethyl)cyclobutan-1-yl]-guanin zur Herstellung eines antiviralen Mittels gegen das Hepatitis B-Virus.

24. Verwendung von 9-[(1R,2R,3S)-2,3-Bis(hydroxymethyl)cyclobutan-1-yl]-guanin zur Herstellung eines antiviralen Mittels gegen ein Human-Immundefekt-Virus.

25. (1R,2R,3S)Cyclobutan-Derivate, dargestellt durch die allgemeine Formel (IV):

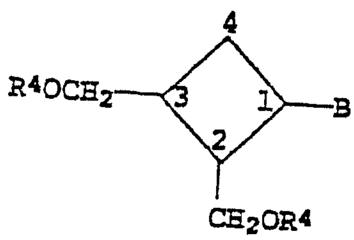

(IV)

worin $R^4$ eine konventionell verwendete Schutzgruppe darstellt und B eine Pyrimidinbase, die dargestellt wird durch

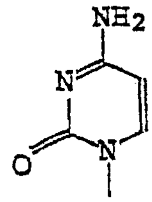 oder 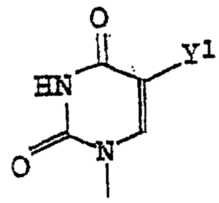

oder ein Purinbase, dargestellt durch

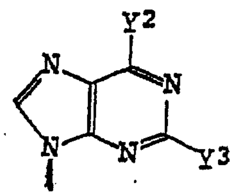 oder 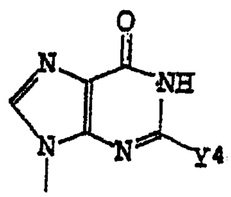

ist, worin $Y^1$ ein Wasserstoffatom oder eine $(C_1$-$C_4)$-Niederalkylgruppe darstellt; $Y^2$ ein Wasserstoffatom, ein Halogenatom oder eine Aminogruppe darstellt; $Y^3$ ein Wasserstoffatom oder eine Aminogruppe darstellt; und $Y^4$ ein Wasserstoffatom oder eine Aminogruppe darstellt.

**26.** (1R,2R,3S)Cyclobutan-Derivate nach Anspruch 25, worin die konventionell verwendete Schutzgruppe von $R^4$ eine Esterschutzgruppe oder eine Etherschutzgruppe ist.

**27.** (1R,2R,3S)Cyclobutan-Derivate nach Anspruch 25, worin die konventionelle Schutzgruppe von $R^4$ eine Acetylgruppe ist.

**Patentansprüche für folgenden Vertragsstaat : ES**

**1.** Verfahren zur Herstellung von (1R,2R,3S)Cyclobutan-Derivaten, dargestellt durch die folgende allgemeine Formel (Z):

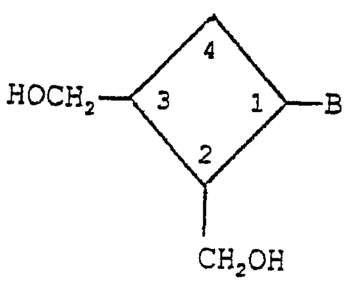 (Z)

worin B eine Pyrimidinbase, dargestellt durch

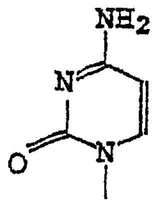 oder 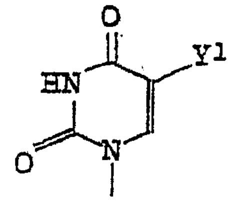

oder eine Purinbase, dargestellt durch

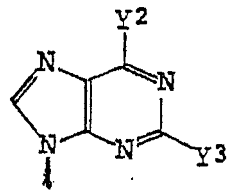 oder 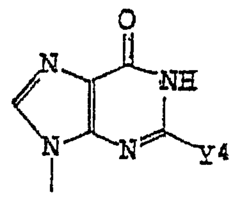

ist, worin $Y^1$ ein Wasserstoffatom oder eine ($C_1$-$C_4$)-Niederalkylgruppe ist; $Y^2$ ein Wasserstoffatom, ein Halogenatom oder eine Aminogruppe ist; $Y^3$ ein Wasserstoffatom oder eine Aminogruppe ist; und $Y^4$ ein Wasserstoffatom oder eine Aminogruppe ist, daß das Umsetzen einer Verbindung, dargestellt durch die folgende allgemeine Formel (V) in einer optisch aktiven Form

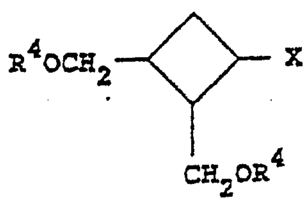 (V)

worin $R^4$ ein Wasserstoff oder eine konventionell verwendete Schutzgruppe darstellt und X eine Abgangsgruppe darstellt, mit der entsprechenden Nucleinsäurebase in einem Lösungsmittel und, wenn die Verbindung, die durch diese Reaktion gebildet wurde, eine Schutzgruppe aufweist, die Eliminierung der erwähnten Schutzgruppe umfaßt.

**2.** Verfahren nach Anspruch 1, worin die konventionell verwendete Schutzgruppe von $R^4$ eine Esterschutzgruppe oder eine Etherschutzgruppe ist.

**3.** Verfahren nach Anspruch 2, worin die erwähnte Esterschutzgruppe eine Acetylgruppe, eine Benzoylgruppe, eine Dimethylcarbamoylgruppe oder eine Diphenylcarbamoylgruppe ist und die erwähnte Etherschutzgruppe eine t-Butyldimethylsilylgruppe, eine t-Butyldiphenylsilylgruppe, eine Methoxymethylgruppe oder eine Benzylgruppe ist.

**4.** Verfahren nach Anspruch 1, worin die Abgangsgruppe eine Sulfonylgruppe oder ein Halogenatom ist.

**5.** Verfahren nach Anspruch 1, worin B eine Purinbase ist.

**6.** Verfahren nach Anspruch 1, worin B Adenin ist.

**7.** Verfahren nach Anspruch 1, worin B Guanin ist.

**8.** Verfahren nach Anspruch 1, worin das (1R,2R,3S)Cyclobutan-Derivat 9-[(1R,2R,3S)-2,3-Bis(hydroxymethyl)cyclobutan-1-yl]-adenin ist.

9. Verfahren nach Anspruch 1, worin das (1R,2R,3S)Cyclobutan-Derivat 9-[(1R,2R,3S)-2,3-Bis(hydroxymethyl)cyclobutan-1-yl]-guanin ist.

10. Verfahren nach Anspruch 1, worin das (1R,2R,3S)Cyclobutan-Derivat 9-[(1R,2R,3S)-2,3-Bis(hydroxymethyl)cyclobutan-1-yl]-hypoxanthin ist.

11. Verfahren nach Anspruch 1, worin das (1R,2R,3S)Cyclobutan-Derivat 2,6-Diamino-9-[(1R,2R,3S)-2,3-bis(hydroxymethyl)cyclobutan-1-yl]-purin ist.

12. Verfahren nach Anspruch 1, worin das (1R,2R,3S)Cyclobutan-Derivat 2-Amino-9-[(1R,2R,3S)-2,3-bis(hydroxymethyl)cyclobutan-1-yl]-6-chloropurin ist.

13. Verfahren nach Anspruch 1, worin das (1R,2R,3S)Cyclobutan-Derivat 2-Amino-9-[(1R,2R,3S)-2,3-bis(hydroxymethyl)cyclobutan-1-yl]-6-purin ist.

14. Verfahren nach Anspruch 1, worin das (1R,2R,3S)Cyclobutan-Derivat 2-Amino-9-[(1R,2R,3S)-2,3-bis(acetoxymethyl)cyclobutan-1-yl]-6-purin ist.

15. Verfahren nach Anspruch 1, worin die Reaktion in einem Lösungsmittel durchgeführt wird in einem Temperaturbereich von 0°C bis zur Rückflußtemperatur des Lösungsmittels.

16. Verfahren zur Herstellung eines (1R,2R,3S)Cyclobutan-Derivates, dargestellt durch die allgemeine Formel (Z)

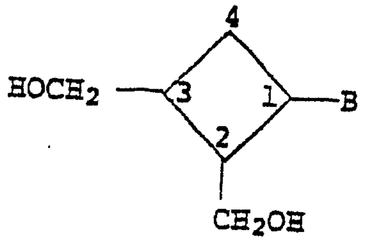

(Z)

worin B eine Pyrimidinbase, dargestellt durch

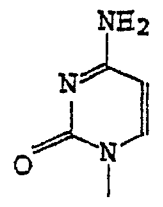

oder

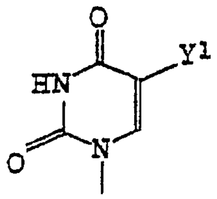

oder eine Purinbase, dargestellt durch

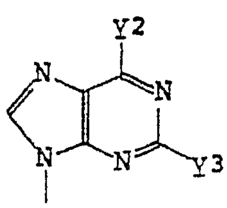

oder

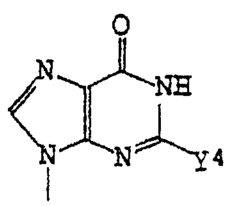

darstellt, worin $Y^1$ ein Wasserstoffatom oder eine $(C_1-C_4)$Niederalkylgruppe darstellt; $Y^2$ ein Wasserstoffatom, ein Halogenatom oder eine Aminogruppe darstellt; $Y^3$ ein Wasserstoffatom oder eine Aminogruppe darstellt und $Y^4$ ein Wasserstoffatom oder eine Aminogruppe darstellt, das die Eliminierung einer Schutzgruppe eines (1R,2R,3S)-Cyclobutan-Derivates, dargestellt durch die folgende allgemeine Formel (IV)

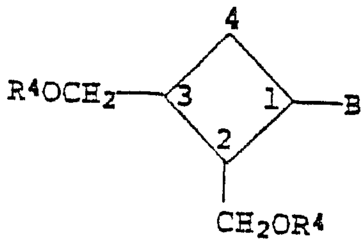

(IV)

worin $R^4$ eine konventionell verwendete Schutzgruppe darstellt und B wie oben definiert ist, umfaßt.

**17.** Verfahren zur Herstellung eines (1R,2R,3S)Cyclobutan-Derivates, dargestellt durch die allgemeine Formel (IV):

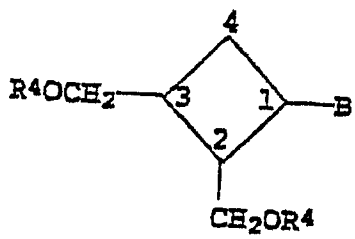

(IV)

worin $R^4$ eine konventionelle Schutzgruppe darstellt und B eine Pyrimidinbase, dargestellt durch

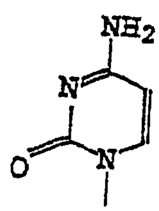

oder

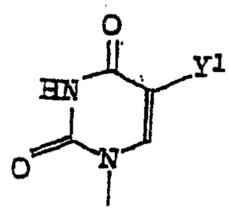

oder eine Purinbase, dargestellt durch

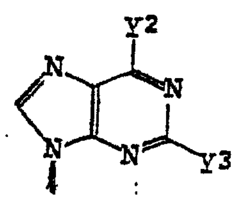

oder

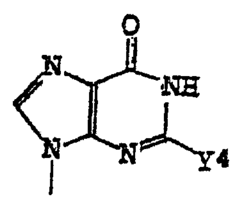

darstellt, worin $Y^1$ ein Wasserstoffatom oder eine ($C_1$-$C_4$)-Niederalkylgruppe ist; $Y^2$ ein Wasserstoffatom, ein Halogenatom oder eine Aminogruppe ist; $Y^3$ ein Wasserstoffatom oder eine Aminogruppe darstellt; und $Y^4$ ein Wasserstoffatom oder eine Aminogruppe darstellt, das die Reaktion einer Verbindung, dargestellt durch die folgende allgemeine Formel (V) in einer optisch aktiven Form

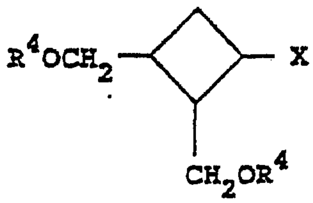

(V)

worin $R^4$ wie oben definiert ist und X eine Abgangsgruppe darstellt, mit der korrespondierenden Nucleinsäurebase in einem Lösungsmittel und Eliminierung der Schutzgruppe umfaßt.

**Revendications**

**Revendications pour les Etats contractants suivants : BE, DE, FR, GB, IT, NL, SE**

1. Dérivé de (1R,2R,3S)-cyclobutane représenté par la formule générale (IV) suivante:

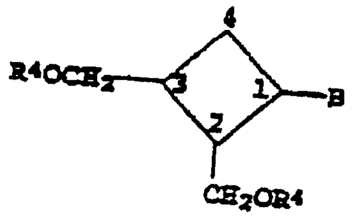

(IV)

dans laquelle $R^4$ représente un atome d'hydrogène et B représente une base pyrimidine représentée par

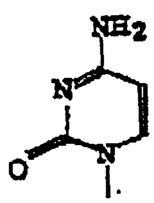

ou

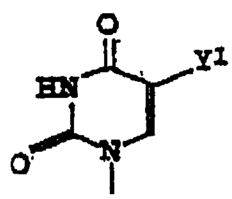

ou une base purine représentée par

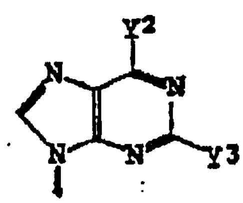

ou

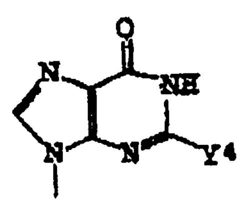

dans laquelle $Y^1$ représente un atome d'hydrogène ou un groupe alkyle inférieur en $C_1$-$C_4$; $Y^2$ représente un atome d'hydrogène, un atome d'halogène ou un groupe amino; $Y^3$ représente un atome d'hydrogène ou un groupe amino; et $Y^4$ représente un atome d'hydrogène ou un groupe amino.

2. Dérivé de (1R,2R,3S)-cyclobutane selon la revendication 1, dans lequel B est la base purine.

3. Dérivé de (1R,2R,3S)-cyclobutane selon la revendication 1, dans lequel B est l'adénine.

4. Dérivé de (1R,2R,3S)-cyclobutane selon la revendication 1, dans lequel B est la guanine.

**5.** 9-[(1R,2R,3S)-2,3-Bis(hydroxyméthyl)cyclobutane-1-yl]adénine.

**6.** 9-[(1R,2R,3S)-2,3-Bis(hydroxyméthyl)cyclobutane-1-yl]guanine.

**7.** 9-[(1R,2R,3S)-2,3-Bis(hydroxyméthyl)cyclobutane-1-yl]hypoxanthine.

**8.** 2,6-Diamino-9-[(1R,2R,3S)-2,3-bis(hydroxyméthyl)cyclobutane-1-yl]purine.

**9.** 2-Amino-9-[(1R,2R,3S)-2,3-bis(acétoxyméthyl)cyclobutane-1-yl]-6-chloropurine.

**10.** 2-Amino-9-[(1R,2R,3S)-2,3-bis(hydroxyméthyl)cyclobutane-1-yl]purine.

**11.** 2-Amino-9-[(1R,2R,3S)-2,3-bis(acétoxyméthyl)cyclobutane-1-yl]purine.

**12.** Agent antiviral comprenant, conune principe actif, un dérivé de (1R,2R,3S)-cyclobutane selon la revendication 1 ou 2.

**13.** Agent antiviral comprenant, conune principe actif, la 9-[(1R,2R,3S)-2,3-bis(hydroxyméthyl)cyclobutane-1-yl]adénine.

**14.** Agent antiviral comprenant, comme principe actif, la 9-[(1R,2R,3S)-2,3-bis(hydroxyméthyl)cyclobutane-1-yl]guanine.

**15.** Agent antiviral selon la revendication 12, 13 ou 14, dans lequel ledit virus est l'herpèsvirus simplex, le cytomégalovirus, le virus de l'hépatite B ou le virus de l'immunodéficience humaine.

**16.** Dérivé de (1R,2R,3S)-cyclobutane selon la revendication 1, à utiliser comme agent antiviral.

**17.** 9-[(1R,2R,3S)-2,3-Bis(hydroxyméthyl)cyclobutane-1-yl]adénine, à utiliser comme agent antiviral.

**18.** 9-[(1R,2R,3S)-2,3-Bis(hydroxyméthyl)cyclobutane-1-yl]guanine, à utiliser comme agent antiviral.

**19.** Utilisation de la 9-[(1R,2R,3S)-2,3-bis(hydroxyméthyl)cyclobutane-1-yl]adénine pour la fabrication d'un agent antiviral contre le virus de l'hépatite B.

**20.** Utilisation de la 9-[(1R,2R,3S)-2,3-bis(hydroxyméthyl)cyclobutane-1-yl]adénine pour la fabrication d'un agent antiviral contre le virus de l'immunodéficience humaine.

**21.** Utilisation de la 9-[(1R,2R,3S)-2,3-bis(hydroxyméthyl)cyclobutane-1-yl]guanine pour la fabrication d'un agent antiviral contre l'herpèsvirus simplex.

**22.** Utilisation de la 9-[(1R,2R,3S)-2,3-bis(hydroxyméthyl)cyclobutane-1-yl]guanine pour la fabrication d'un agent antiviral contre le cytomégalovirus.

**23.** Utilisation de la 9-[(1R,2R,3S)-2,3-bis(hydroxyméthyl)cyclobutane-1-yl]guanine pour la fabrication d'un agent antiviral contre le virus de l'hépatite B.

**24.** Utilisation de la 9-[(1R,2R,3S)-2,3-bis(hydroxyméthyl)cyclobutane-1-yl]guanine pour la fabrication d'un agent antiviral contre le virus de l'immunodéficience déficiente humaine.

**25.** Dérivé de (1R,2R,3S)-cyclobutane représenté par la formule générale (IV) suivante:

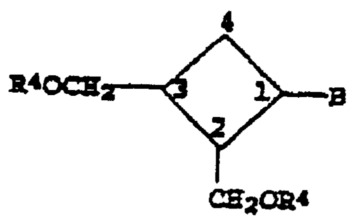

(IV)

dans laquelle $R^4$ représente un groupe protecteur couramment utilisé et B représente une base pyrimidine représentée par

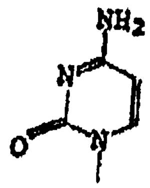

ou

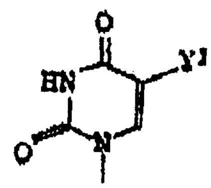

ou une base purine représentée par

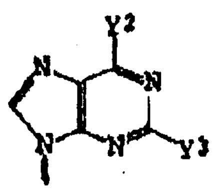

ou

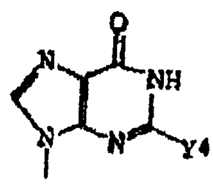

dans laquelle $Y^1$ représente un atome d'hydrogène ou un groupe alkyle inférieur en $C_1$-$C_4$; $Y^2$ représente un atome d'hydrogène, un atome d'halogène ou un groupe amino; $Y^3$ représente un atome d'hydrogène ou un groupe amino; et $Y^4$ représente un atome d'hydrogène ou un groupe amino.

**26.** Dérivé de (1R,2R,3S)-cyclobutane selon la revendication 25, dans lequel le groupe protecteur couramment utilisé pour $R^4$ est un groupe protecteur de type ester ou un groupe protecteur de type éther.

**27.** Dérivé de (1R,2R,3S)-cyclobutane selon la revendication 25, dans lequel le groupe protecteur couramment utilisé pour $R^4$ est un groupe acétyle.

**Revendications pour l'Etat contractant suivant : ES**

**1.** Procédé de production d'un dérivé de (1R,2R,3S)-cyclobutane représenté par la formule générale (Z) suivante:

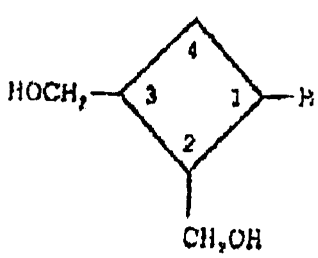

(2)

dans laquelle B représente une base pyrimidine représentée par

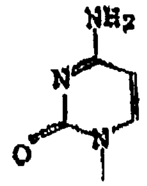 ou 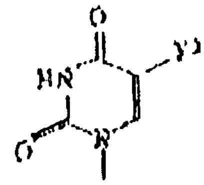

ou une base purine représentée par

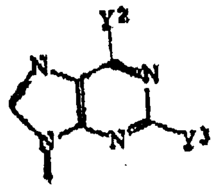 ou 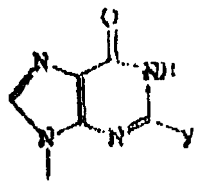

dans laquelle $Y^1$ représente un atome d'hydrogène ou un coupe alkyle inférieur en $C_1$-$C_4$; $Y^2$ représente un atome d'hydrogène, un atome d'halogène ou un groupe amino; $Y^3$ représente un atome d'hydrogène ou un groupe amino; et $Y^4$ représente un atome d'hydrogène ou un groupe amino, qui comprend la réaction d'un composé représenté par la formule générale (V) suivante, sous une forme optiquement active,

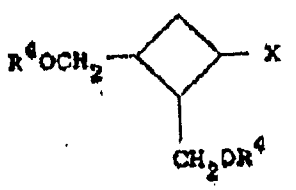

(V)

dans laquelle $R^4$ représente un atome d'hydrogène ou un groupe protecteur couramment utilisé et X représente un groupe partant, avec une base d'acide nucléique correspondante dans un solvant et, lorsque le composé formé par cette réaction porte un groupe protecteur, l'élimination dudit groupe protecteur.

2. Procédé selon la revendication 1, dans lequel le groupe protecteur couramment utilisé pour $R^4$ est un groupe protecteur de type ester ou un groupe protecteur de type éther.

3. Procédé selon la revendication 2, dans lequel ledit groupe protecteur de type ester est un groupe acétyle, un groupe benzoyle, un groupe diméthylcarbamoyle ou un groupe diphénylcarbamoyle, et ledit groupe protecteur de type éther est un groupe t-butyldiméthylsilyle, un groupe t-butyldiphénylsilyle, un groupe méthoxylméthyle ou un groupe benzyle.

4. Procédé selon la revendication 1, dans lequel ledit groupe partant est un groupe sulfonyloxy ou un atome d'halogène.

5. Procédé selon la revendication 1, dans lequel B est la base purine.

6. Procédé selon la revendication 1, dans lequel B est l'adénine.

7. Procédé selon la revendication 1, dans lequel B est la guanine.

8. Procédé selon la revendication 1, dans lequel le dérivé de (1R,2R,3S)-cyclobutane est la 9-[(1R,2R,3S)-2,3-bis(hydroxyméthyl)-cyclobutane-1-yl]adénine.

9. Procédé selon la revendication 1, dans lequel le dérivé de (1R,2R,3S)-cyclobutane est la 9-[(1R,2R,3S)-2,3-bis(hydroxyméthyl)-cyclobutane-1-yl]guanine.

**10.** Procéde selon la revendication 1, dans lequel le dérivé de (1R,2R,3S)-cyclobutane est la 9-[(1R,2R,3S,)-2,3-bis(hydroxyméthyl)-cyclobutane-1-yl]hypoxanthine.

**11.** Procédé selon la revendication 1, dans lequel le dérivé de (1R,2R,3S)-cyclobutane est la 2,6-diamino-9-[(1R,2R,3S,)-2,3-bis(hydroxyméthyl)cyclobutane-1-yl]purine.

**12.** Procédé selon la revendication 1, dans lequel le dérivé de (1R,2R,3S)-cyclobutane est la 2-amino-9-[(1R,2R,3S,)-2,3-bis(hydroxyméthyl)cyclobutane-1-yl]-6-chloropurine.

**13.** Procédé selon la revendication 1, dans lequel le dérivé de (1R,2R,3S)-cyclobutane est la 2-amino-9-[(1R,2R,3S,)-2,3-bis(hydroxyméthyl)cyclobutane-1-yl]purine.

**14.** Procédé selon la revendication 1, dans lequel le dérivé de (1R,2R,3S)-cyclobutane est la 2-amino-9-[(1R,2R,3S,)-2,3-bis(acétoxyméthyl)cyclobutane-1-yl]purine.

**15.** Procédé selon la revendication 1, dans lequel la réaction est réalisée dans un solvant à une température s'échelonnant de 0°C à la température de reflux du solvant.

**16.** Procédé de production d'un dérivé de (1R,2R,3S)-cyclobutane représenté par la formule générale (Z)

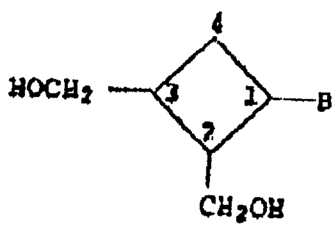

(Z)

dans laquelle B représente une base pyrimidine représentée par

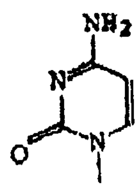

ou

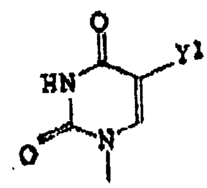

ou une base purine représentée par

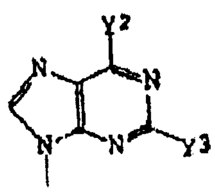

ou

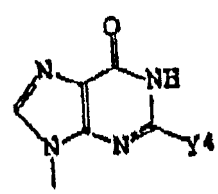

dans laquelle $Y^1$ représente un atome d'hydrogène ou un groupe alkyle inférieur en $C_1$-$C_4$; $Y^2$ représente un atome d'hydrogène, un atome d'halogène ou un groupe amino; $Y^3$ représente un atome d'hydrogène ou un groupe amino; et $Y^4$ représente un atome d'hydrogène ou un groupe amino, qui comprend l'élimination d'un groupe protecteur du dérivé de (1R,2R,3S)-cyclobutane représenté par la formule générale (IV) suivante:

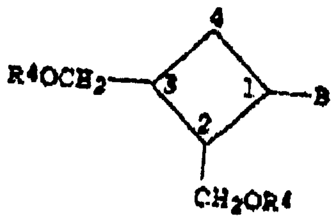

(IV)

dans laquelle $R^4$ représente un groupe protecteur couramment utilisé et B est tel que défini ci-dessus.

**17.** Procédé de production d'un dérivé de (1R,2R,3S)-cyclobutane représenté par la formule générale (IV) suivante:

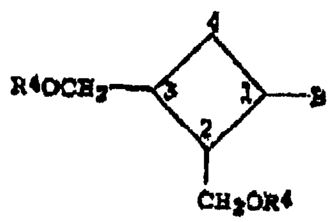

(IV)

dans laquelle $R^4$ représente un groupe protecteur couramment utilisé et B représente une base pyrimidine représentée par

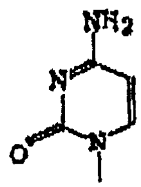

ou

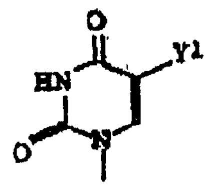

ou une base purine représentée par

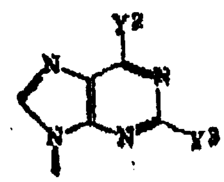

ou

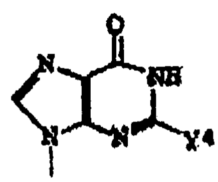

dans laquelle $Y^1$ représente un atome d'hydrogène ou un groupe alkyle inférieur en $C_1$-$C_4$; $Y^2$ représente un atome d'hydrogène, un atome d'halogène ou un groupe amino; $Y^3$ représente un atome d'hydrogène ou un groupe amino; et $Y^4$ représente un atome d'hydrogène ou un groupe amino, qui comprend la réaction d'un composé représenté par la formule générale (V) suivante, sous une forme optiquement active,

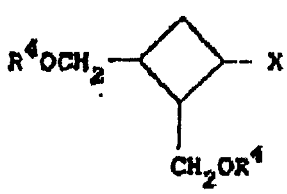

(V)

dans laquelle $R^4$ est tel que défini ci-dessus et X représente un groupe partant, avec une base d'acide nucléique

correspondante dans un solvant et l'élimination dudit groupe protecteur.